# EUROPÄISCHE PATENTSCHRIFT

(11) **EP 1 631 537 B1**
(45) Veröffentlichungstag und Bekanntmachung des Hinweises auf die Patenterteilung: **21.09.2011**
(21) Anmeldenummer: 04730988.5
(22) Anmeldetag: 04.05.2004
(51) Int. Cl.: C07C 59/105, C07H 7/027, C07C 51/235

(54) **VERFAHREN ZUR SELEKTIVEN KOHLENHYDRAT-OXIDATION UNTER VERWENDUNG GETRÄGERTER GOLD-KATALYSATOREN**
METHOD FOR SELECTIVE CARBOHYDRATE OXIDATION USING SUPPORTED GOLD CATALYSTS
PROCEDE D'OXYDATION SELECTIVE D'UN HYDRATE DE CARBONE AU MOYEN DE CATALYSEURS A BASE D'OR SUPPORTES

(30) Priorität: 05.05.2003 DE 10319917
(43) Veröffentlichungstag der Anmeldung: 08.03.2006
(60) Teilanmeldung: 10011677.1
(73) Patentinhaber: Südzucker Aktiengesellschaft Mannheim/Ochsenfurt, 68165 Mannheim (DE)
(72) Erfinder: KOWALCZYK, Jörg, 67304 Eisenberg/Steinborn (DE); HAJI BEGLI, Alireza, 67305 Ramsen (DE); PRÜSSE, Ulf, 38118 Braunschweig (DE); BERNDT, Heinz, 12623 Berlin (DE); PITSCH, Irene, 12487 Berlin (DE)
(74) Vertreter: Schwahn, Hartmut
(86) Internationale Anmeldenummer: PCT/EP2004/004703
(87) Internationale Veröffentlichungsnummer: WO 2004/099114

(56) Entgegenhaltungen:
- DE-A- 4 307 388
- DE-A- 19 804 709
- DE-C- 3 823 301
- US-A- 4 985 553
- BIELLA S ET AL: "Selective Oxidation of D-Glucose on Gold Catalyst" JOURNAL OF CATALYSIS, ACADEMIC PRESS, DULUTH, MN, US, Bd. 206, Nr. 2, 10. März 2002 (2002-03-10), Seiten 242-247, XP004445070 ISSN: 0021-9517 in der Anmeldung erwähnt

## Beschreibung

Die vorliegende Erfindung betrifft ein Verfahren zur selektiven Oxidation eines Kohlenhydrates oder eines Kohlenhydrat-Gemisches in Gegenwart eines Gold-Katalysators, der nanodispers verteilte Gold-Partikel auf einem Metalloxid-Träger umfasst, sowie die Verwendung dieses Katalysators dafür.

Bei der Käse-Produktion und der Molkeverarbeitenden Industrie fallen jährlich weltweit etwa 1,2 Mio. Tonnen Lactose als Abfallprodukt an. Lactose als wichtigster Kohlenhydrat-Bestandteil der Milch hat bislang jedoch kaum wirtschaftliche Bedeutung. Einer der Gründe dafür ist in der Lactose-Intoleranz von Teilen der Bevölkerung zu suchen. Personen mit Lactose-Intoleranz können Lactose nicht verwerten und reagieren bei Lactose-Konsum mit Unverträglichkeitssymptomen wie Durchfall. Nur ein relativ geringer Teil der anfallenden Lactose wird einer wirtschaftlichen Verwertung zugeführt, wobei Lactose beispielsweise als Fermentationssubstrat, als Füllmittel oder zur Herstellung von Diät-Lebensmitteln eingesetzt wird. Der größte Teil der anfallenden Lactose-Mengen wird jedoch über die Abwasseranlagen der Produzenten entsorgt, was zu Störungen des ökologischen Gleichgewichtes in Gewässern führen kann. Da Lactose jedoch ein in großen Mengen vorhandener und äußerst kostengünstiger Rohstoff ist, besteht großes Interesse an einer wirtschaftlichen Aufwertung dieses Kohlenhydrates. In jüngster Zeit wurden daher insbesondere verschiedene enzymatische Spaltungs- und Transformationsreaktionen entwickelt, bei denen Lactose als Ausgangsmaterial zur Herstellung höherwertige Produkte eingesetzt wird.

Durch Oxidation von Lactose kann so Lactobionsäure erhalten werden, welche für eine Reihe von Anwendungen äußerst interessant ist. Bislang wird zur Lactobionsäure-Herstellung aus Lactose ein enzymatisches Verfahren unter Verwendung der Enzyme Cellobiose-Dehydrogenase und Hexose-Oxidase eingesetzt. Der nicht zufriedenstellende Umsatz beispielsweise bei der Cellobiose-Dehydrogenasekatalysierten Reaktion lässt sich unter Verwendung des Enzyms Laccase steigern, das die bei der Reaktion reduzierten Redox-Mediatoren reoxidiert. Aufgrund ihrer exzellenten Fähigkeit, Metallchelate zu bilden, wird Lactobionsäure unter anderem in der sogenannten "Wisconsin-Transplantations-Lösung" eingesetzt, da Lactobionsäure den durch Metallionen bedingten oxidativen Schaden während der Lagerung von zu transplantierenden Organen reduzieren kann. Lactobionsäure lässt sich ebenfalls als biologisch abbaubarer Cobuilder in Waschpulver einsetzen, das bis zu 40 % Lactobionsäure enthalten kann. Aufgrund des milden süßlich-sauren Geschmacks von Lactobionsäure bestehen weitere Anwendungsmöglichkeiten in der Lebensmitteltechnologie.

Auch für andere Aldonsäuren beziehungsweise Oligosaccharid-Aldonsäuren besteht ein großes Einsatzpotential in der pharmazeutischen Industrie, der Kosmetik-Herstellung und in der Lebensmitteltechnologie. Aldonsäuren werden derzeit hauptsächlich mittels mikrobieller oder enzymatischer Umwandlung aus den entsprechenden Mono- oder Oligosacchariden hergestellt. So kann beispielsweise Glucose unter Verwendung von Acetobacter methanolicus in Gluconsäure umgewandelt werden. Die enzymatische Herstellung von Aldonsäuren ist jedoch generell durch eine relativ geringe Produktivität gekennzeichnet und auch aus Gründen des Umweltschutzes nicht unproblematisch. Daher besteht ein großes Interesse an alternativen Oxidationsverfahren, die zu einer deutlich geringeren Belastung der Umwelt führen, wobei das zu oxidierende Kohlenhydrat, beispielsweise ein Monosaccharid, unter Verwendung eines heterogenen Katalysators zu der entsprechenden Aldonsäure oxidiert wird.

Die heterogene Katalyse einer Oxidationsreaktion erfolgt üblicherweise in einem Drei-Phasen-Reaktor, wobei der feste, meist pulverförmige Katalysator in einer die zu oxidierende Verbindung enthaltenden Flüssigphase suspendiert ist und während der Reaktion Sauerstoff durch die Flüssigphase hindurchgeperlt wird. Obwohl die katalytische Oxidation im Vergleich zur enzymatischen Umsetzung einige erhebliche Vorteile bietet, insbesondere unter dem Gesichtspunkt der erheblich geringeren Umweltverschmutzung, weist sie jedoch einen entscheidenden Nachteil auf. Bei Verwendung von Metallen kann die Disauerstoff-Aktivierung zu radikalischen Reaktionen führen, die insbesondere im Fall polyfunktionaler Moleküle die Selektivität der Umsetzung deutlich senken können (Sheldon und Kochi, "Metal Catalyzed Oxidations of Organic Compounds", 1981, Academic Press, New York).

Bislang am besten untersucht ist die Verwendung geträgerter Palladium- und Platin-Katalysatoren zur Oxidation von Glucose. Dabei hat sich herausgestellt, dass bei Verwendung dieser Katalysatoren die katalytische Umsetzung von Glucose zu Gluconsäure aufgrund der geringen Selektivität und Umsetzungsrate erheblich eingeschränkt ist. Auch kommt es zu einer relativ schnellen Deaktivierung beider Katalysator-Typen. Diese Deaktivierung beruht offensichtlich entweder auf einer Blockierung der Katalysator-Oberfläche aufgrund der Adsorption von Molekülen oder auf Disauerstoff-bedingten Vergiftungseffekten (Van Dam, Kieboom und van Bekkum, Appl. Catal., 33 (1990), 187). Einige der Faktoren, die die katalytische Umsetzung von Glucose zu Gluconsäure einschränken, lassen sich durch die Einführung von Promotoren wie Bismut oder Blei deutlich-verbessern. Neben einer Verbesserung der Katalysator-Lebensdauer erhöhen sich dadurch insbesondere die Selektivität und die Umsetzungsrate der Reaktion (Fiege und Wedemeyer, Angew. Chem., 93 (1981), 812; Wenkin et al., Appl. Catal. A: General, 148 (1996), 181).

Pd und Bi werden aufgrund eines möglichen Leachings dieser toxikologisch bedenklichen Substanzen jedoch kontrovers diskutiert. Zur Erhöhung der Umsetzungsgeschwindigkeit und zur Vermeidung der Katalysator-Deaktivierung sind leicht alkalische Bedingungen erforderlich. Unter solchen Bedingungen treten jedoch Nebenreaktionen auf, die die Gluconat-Produktion vermindern. Ebenfalls nachteilig ist, dass bei Verwendung von Basen anstelle der freien Gluconsäure Gluconat erzeugt wird (Biella, Prati und Rossi, Journal of Catalysis, 206 (2002), 242-247).

Zur großtechnischen Produktion von Gluconsäure wird daher nach wie vor dem Fermentationsprozess der Vorrang gegeben, trotz der bei diesem Verfahren auftretenden Probleme, beispielsweise der starken Abwasser-Verunreinigung und der nicht unerheblichen Bildung von Nebenprodukten. Aus diesem Grund ist es erforderlich, neue Katalysator-Typen zu entwickeln, die eine katalytische Oxidation von Kohlenhydraten zur Herstellung von Aldonsäuren unter Verwendung von Disauerstoff als Oxidationsmittel ermöglichen und neben hoher Aktivität und Selektivität eine lange Lebensdauer aufweisen.

Bislang wurden geträgerte Gold-Katalysatoren vor allem zur Oxidation von CO oder Propen in der Gasphase und für Selektivhydrierungen eingesetzt. Biella et al., Journal of Catalysis, 206 (2002) 242-247, beschreiben die Verwendung eines Kohlenstoff-geträgerten Gold-Katalysators zur selektiven Oxidation von D-Glucose zu D-Gluconsäure in der Flüssigphase. Ein Vergleich zwischen dem Kohlenstoff-geträgerten Gold-Katalysator und herkömmlichen Palladium- und Platin-Katalysatoren zeigt, dass der Gold-Katalysator sowohl Palladium- als auch Platin-Katalysatoren in mehrfacher Hinsicht überlegen ist. Im Vergleich zu Palladium- und Platin-Katalysatoren erweist sich der verwendete Gold-Katalysator insbesondere wesentlich stabiler gegenüber einer Deaktivierung. Ein weiterer Vorteil des verwendeten Gold-Katalysators besteht darin, dass dieser bei der Glucose-Umsetzung keine externe pH-Kontrolle erfordert. Die verwendeten Kohlenstoff-geträgerten Gold-Katalysatoren weisen jedoch einen erheblichen Nachteil auf. Einerseits wird mit abnehmendem pH-Wert vermehrt Gold aus dem Katalysator ausgelaugt. Andererseits wird mit steigendem pH-Wert das Wachstum der Goldpartikel gefördert. Beides führt zu einer Abnahme der Katalysator-Aktivität. Das mit steigendem pH-Wert zunehmende Auflösen von Goldpartikeln geht mit einer Vergrößerung der Goldpartikel einher. Dies ist wahrscheinlich darauf zurückzuführen, dass kleine Goldpartikel in Lösung gehen und sich dann das Gold an größeren Gold-Partikeln abscheidet, wobei es zu einer Reduktion von Au(I,III)-Partikeln kommt.

Das der vorliegenden Erfindung zugrunde liegende technische Problem besteht darin, gegenüber den im Stand der Technik bekannten Katalysatoren verbesserte Gold-Katalysatoren bereitzustellen, die eine hohe Selektivität und Aktivität bezüglich der Oxidation von Kohlenhydraten zu den entsprechenden Aldonsäuren aufweisen und bei denen die im Stand der Technik bekannten Probleme von Gold-Katalysatoren, insbesondere der durch mehrfache Verwendung bedingte Aktivitätsabfall, dessen Ausmaß vom pH-Wert abhängig ist, nicht auftreten, so dass die Gold-Katalysatoren für die großtechnische Aldonsäure-Herstellung aus geeigneten Kohlenhydrat-Ausgangsmaterialien eingesetzt werden können. Darüber hinaus sollen die verbesserten Gold-Katalysatoren für die Oxidation einer Vielzahl unterschiedlicher Kohlenhydrate zu den entsprechenden Aldonsäuren eingesetzt werden können.

Die vorliegende Erfindung löst das ihr zugrunde liegende technische Problem durch das Verfahren gemäß Anspruch 1.

Die erfindungsgemäß zur Kohlenhydrat-Oxidation eingesetzten Metalloxid-geträgerten Gold-Katalysatoren zeichnen sich dadurch aus, dass sie die selektive Oxidation einer Vielzahl von Kohlenhydraten, insbesondere solchen Kohlenhydraten, deren C1-Kohlenstoffatom eine oxidierbare Aldehyd-Gruppe aufweist beziehungsweise mit einer oxidierbaren Aldehyd-Gruppe versehen werden kann, zu den entsprechenden Aldonsäuren ermöglichen. Gegenüber herkömmlichen Platin- und Palladiumkatalysatoren weisen die erfindungsgemäß verwendeten Gold-Katalysatoren eine bis zu einer Größenordnung höhere Aktivität bezüglich der selektiven Kohlenhydrat-Oxidation auf. Diese hohe Aktivität ist umso bemerkenswerter, als die Metallbeladung der Gold-Katalysatoren im Vergleich zu Platin- oder Palladium-Katalysatoren erheblich geringer ist. Darüber hinaus weisen die erfindungsgemäß verwendeten Metalloxid-geträgerten Gold-Katalysatoren gegenüber Platin- und Palladium-Katalysatoren eine erheblich gesteigerte Selektivität bezüglich der Aldonsäure-Produktion auf, das heißt, dass die Selektivität auf andere Oxidationsprodukte deutlich reduziert ist. Während bei Verwendung von Platin- und Palladium-Katalysatoren stets ein Produkt-Gemisch erhalten wird, dass neben der gewünschten Aldonsäure eine Vielzahl anderer Oxidationsprodukte enthält, die auf die Oxidation von beispielsweise Alkohol-Gruppen zurückgehen, wird unter Verwendung der erfindungsgemäß eingesetzten Metalloxid-geträgerten Gold-Katalysatoren eine nahezu reine Aldonsäure erhalten, die keine anderen nachweisbaren Oxidationsprodukte oder nur äußerst geringe Mengen davon enthält. Die außerordentlich hohe Selektivität der erfindungsgemäß eingesetzten Metalloxid-geträgerten Gold-Katalysatoren ist vor allem darauf zurückzuführen, dass nur die Aldehyd-Gruppe des C1-Kohlenstoffatoms oxidiert wird, nicht jedoch Alkohol-Gruppen.

Gegenüber Kohlenstoff-geträgerten Gold-Katalysatoren zeichnen sich die erfindungsgemäß eingesetzten Metalloxid-geträgerten Gold-Katalysatoren durch eine erheblich längere Lebensdauer aus. Während bei Kohlenstoff-geträgerten Gold-Katalysatoren bereits nach ein- oder zweimaliger Verwendung eine relativ deutliche Aktivitätsabnahme beobachtet wurde (Biella et al., J. Catalysis, 206 (2002), 242-247), lassen sich die Metalloxid-geträgerten Gold-Katalysatoren mindestens 20-mal verwenden, ohne dass die Katalysator-Aktivität und Selektivität der Oxidation in nennenswertem Umfang abnehmen. Die erfindungsgemäß eingesetzten Metalloxid-geträgerten Katalysatoren lassen sich daher in vorteilhafter Weise zur großtechnischen Herstellung von Aldonsäuren einsetzen. Ein weiterer Vorteil der erfindungsgemäß eingesetzten Metalloxid-geträgerten Gold-Katalysatoren besteht darin, dass sie zur Oxidation einer Vielzahl unterschiedlicher Kohlenhydrat-Ausgangssubstrate, beispielsweise Monosaccharide, Oligosaccharide oder Gemische davon, eingesetzt werden können. Vorteilhafterweise können sowohl Aldosen als auch Ketosen oxidiert werden.

Im Zusammenhang mit der vorliegenden Erfindung wird unter einem "Katalysator" ein Stoff verstanden, der die zum Ablauf einer bestimmten Reaktion benötigte Aktivierungsenergie herabsetzen und dadurch die Reaktionsgeschwindigkeit erhöhen kann, ohne im Endprodukt der Reaktion zu erscheinen. Unter einem "Gold-Katalysator" wird ein Katalysator verstanden, der Gold auf einem Trägermaterial umfasst oder aus Gold auf einem Trägermaterial besteht, wobei das Gold in Form von dispers verteilten Nanopartikeln auf dem Träger vorliegt. Die Gold-Nanopartikel weisen einen Durchmesser von weniger als 20 nm, vorzugsweise < 10 nm und besonders bevorzugt < 5 nm auf.

Unter einem "Metalloxid-Träger" wird im Zusammenhang mit der vorliegenden Erfindung ein Katalysator-Träger verstanden, der aus mindestens einem Oxid eines Haupt- oder Nebengruppen-Metalls oder - Halbmetalls einschließlich Verbindungen, die mehr als ein Metall und/oder Halbmetall umfassen, besteht. Vorzugsweise handelt es sich dabei um Oxide von Metallen oder Halbmetallen der 2. Hauptgruppe des PSE wie MgO, CaO oder BaO, Oxide von Metallen oder Halbmetallen der 3. Hauptgruppe des PSE wie Al₂O₃ oder Lanthanoid-Oxide oder Oxide von Metallen oder Halbmetallen der 4. Hauptgruppe des PSE wie SiO₂ TiO₂ SnO₂ oder ZrO₂. Vorzugsweise handelt es sich bei den Verbindungen mit mehr als einem Metall und/oder Halbmetall um Silikate, insbesondere Alumosilikate.

Eine bevorzugte Ausführungsform der Erfindung betrifft die Verwendung eines Gold-Katalysators zur Oxidation von Kohlenhydraten, wobei der Katalysator nanodispers verteilte Gold-Partikel auf einem Al₂O₃-Träger umfasst. Erfindungsgemäß enthält der Al₂O₃₋geträgerte Gold-Katalysator etwa 0,1 % bis 5 % Gold, vorzugsweise etwa .0,5 % bis 1 % Gold. Der erfindungsgemäß zur selektiven Oxidation von Kohlenhydraten eingesetzte Al₂O₃-geträgerte Gold-Katalysator enthält vorzugsweise Gold-Nanopartikel mit einem Durchmesser von < 10 nm, besonders bevorzugt < 6 nm und am bevorzugtesten von 1 bis 2 nm.

Eine bevorzugte Ausführungsform der Erfindung betrifft die Verwendung eines Gold-Katalysators zur Oxidation von Kohlenhydraten, wobei der Katalysator nanodispers verteilte Gold-Partikel auf einem TiO₂-Träger umfasst. Erfindungsgemäß enthält der TiO₂-geträgerte Gold-Katalysator etwa 0,1 % bis 5 % Gold, vorzugsweise etwa 0,5 % bis 1 % Gold, besonders bevorzugt 0,5 % Gold. Der erfindungsgemäß zur selektiven Kohlenhydrat-Oxidation verwendete TiO₂-geträgerte Gold-Katalysator enthält vorzugsweise Gold-Nanopartikel mit einem Durchmesser von < 20 nm, besonders bevorzugt < 6 nm und am bevorzugtesten von 1 -4 nm.

Zur Herstellung der erfindungsgemäß verwendeten Metalloxid-geträgerten Gold-Katalysatoren, das heißt zur Abscheidung von Gold in Form von Nanopartikeln auf den Metalloxid-Trägermaterialien, können beispielsweise Fällungsverfahren eingesetzt werden, wobei das Gold in Form von oxidischen/hydroxischen Gold-Präcursoren durch Fällung auf den Metalloxid-Träger abgeschieden oder gemeinsam mit einem Präcursor des Metalloxid-Trägers gefällt wird. Au kann auch in der Sol-Gel-Synthese des Trägers, beispielsweise eines Erdmetall- oder Übergansmetalloxids, eingebracht werden. Bekannt sind ebenfalls das Imprägnieren mit Gold-Lösungen und die Trägerung von Au-Kolloiden unter Verwendung verschiedener Polymere als Kolloid-Stabilisatoren. Geeignete Verfahren zur Herstellung Metalloxid-geträgerter Gold-Katalysatoren umfassen beispielsweise Präzipitationsverfahren, Abscheidungs-PräzipitationsVerfahren und Verfahren zur chemische Abscheidung aus der Gasphase (CVD-Verfahren) und sind unter anderem in Prati und Martra, Gold Bulletin, 32(3) (1999), 96-101; Wolf und Schüth, Applied Catalysis A: General., 226 (2002), 1-13, sowie Berndt et al., Applied Catalysis A: General, 6442 (2003), 1-11, beschrieben, wobei der Offenbarungsgehalt dieser Druckschriften durch Bezugnahme vollständig in den Offenbarungsgehalt der vorliegenden Erfindung einbezogen wird.

Erfindungsgemäß wird der zur Kohlenhydrat-Oxidation verwendete Metalloxid-geträgerte Katalysator in einer heterogenen Katalyse eingesetzt. Das heißt, der Katalysator liegt als Feststoff vor, während die zu oxidierenden Kohlenhydrate in Flüssigphase, beispielsweise als wässrige Lösung, vorliegen. Der zur Kohlenhydrat-Oxidation eingesetzte Disauerstoff wird dann als Gas durch die flüssige Phase hindurchgeperlt und durch intensives Rühren in der Flüssigphase verteilt und gelöst.

Zur erfindungsgemäßen selektiven Oxidation von Kohlenhydraten, Kohlenhydrat-Gemischen oder diese(s) enthaltenden Zusammensetzungen wird der Metalloxid-geträgerte Gold-Katalysator vorzugsweise in Form eines Pulvers oder Granulats eingesetzt.

Unter der "selektiven Oxidation von Kohlenhydraten" wird insbesondere die Oxidation einer oxidierbaren Aldehyd-Gruppe am C1-Kohlenstoffes eines Kohlenhydrats zu einer Carboxyl-Gruppe verstanden, wohingegen Alkohol-Gruppen an anderen Kohlenstoffatomen des Kohlenhydrates nicht oxidiert werden. Im Ergebnis der erfindungsgemäßen selektiven Oxidation des Kohlenhydrates wird daher vorzugsweise eine Aldonsäure erhalten. Im Zusammenhang mit der vorliegenden Erfindung wird unter einer "Aldonsäure" eine Zuckersäure verstanden, die durch Oxidation einer Aldehyd-Gruppe eines Kohlenhydrates zur Carboxyl-Gruppe erhalten wird. Aldonsäuren können unter Wasserabspaltung γ- oder δ-Lactone bilden. Freie Aldonsäuren liegen mit Lactonen im Gleichgewicht vor. Im Zusammenhang mit der vorliegenden Erfindung werden unter "Kohlenhydraten" Polyhydroxyaldehyde und Polyhydroxyketone sowie höhermolekulare Verbindungen verstanden, die sich durch Hydrolyse in solche Verbindungen überführen lassen. Der Begriff "Kohlenhydrat" umfasst auch Derivate, also Abkömmlinge eines Kohlenhydrates, die aus dem Kohlenhydrat in ein oder mehreren Reaktionsschritten gebildet werden. Bei den erfindungsgemäß eingesetzten Kohlenhydraten handelt es sich vorzugsweise um Aldosen, die am C1-Kohlenstoff eine oxidierbare Aldehyd-Gruppe aufweisen, oder um 2-Ketosen, bei denen am C1-Kohlenstoffatom eine oxidierbare Aldehyd-Gruppe eingeführt werden kann.

Im Zusammenhang mit der vorliegenden Erfindung wird unter einer "Aldose" ein Kohlenhydrat verstanden, das eine Aldehyd-Gruppe (-CHO) und mindestens ein Asymmetriezentrum besitzt, wobei die Nummerierung der Aldose-Kette an dem Kohlenstoffatom, dass die Aldehyd-Gruppe enthält, beginnt. Durch die selektive Oxidation der Aldehyd-Gruppe einer Aldose wird eine Aldonsäure erhalten. Bei der selektiven Oxidation eines Gemisches von Aldosen wird daher ein Gemisch unterschiedlicher Aldonsäuren erhalten.

Die vorliegende Erfindung betrifft daher ein Verfahren zur Herstellung einer Aldonsäure oder eines Gemisches verschiedener Aldonsäuren durch selektive Oxidation einer oder mehrerer Aldosen mit einer oxidierbaren Aldehyd-Gruppe unter Verwendung eines Metalloxid-geträgerten Gold-Katalysators.

Im Zusammenhang mit der vorliegenden Erfindung wird unter einer "Ketose" ein Kohlenhydrat verstanden, dass eine Keto-Gruppe enthält. Befindet sich die Keto-Gruppe in 2-Stellung, handelt es sich um eine 2-Ketose. Ketosen zeigen infolge der Bildung von Anomeren Mutarotation. Obwohl sich Ketosen im Allgemeinen nicht oxidieren lassen, können 2-Ketosen in Aldosen überführt werden, wobei die 2-Ketose zunächst in die entsprechende Enol-Form überführt wird, die dann zu einer oxidierbaren Aldose isomerisiert wird, wobei die -OH-Gruppe am C₁-Kohlenstoffatom zur Carbonyl-Gruppe wird und die ursprünglich am C2-Kohlenstoffatom vorhandene C=O-Gruppe zu einer HC-OH-Gruppe wird. Dabei verschiebt sich rein formal betrachtet die Carbonyl-Gruppe von der C₂- auf die C₁-Position.

Die vorliegende Erfindung betrifft daher auch ein Verfahren zur Herstellung einer Aldonsäure oder eines Gemisches verschiedener Aldonsäuren unter Verwendung einer oder mehrerer 2-Ketosen, wobei die 2-Ketose(n) zunächst in die tautomere(n) Aldose-Form(en) mit einer oxidierbaren Aldehyd-Gruppe überführt und dann unter Verwendung eines Metalloxid-geträgerten Gold-Katalysators selektiv oxidiert wird/werden.

Erfindungsgemäß umfassen die zu oxidierenden Kohlenhydrate sowohl monomere Polyhydroxyaldehyde oder Polyhydroxyketone, also Monosaccharide, deren Dimere bis Dekamere, das heißt Oligosaccharide wie Disaccharide, Trisaccharide etc., und die makromolekularen Polysaccharide. Unter "Monosacchariden" werden im Zusammenhang mit der vorliegenden Erfindung Verbindungen der allgemeinen chemischen Formel CₙH₂ₙOₙ mit 2 bis 6 Sauerstoff-Funktionen verstanden, wobei natürliche Monosaccharide im Wesentlichen Hexosen und Pentosen sind. Die KohlenstoffKette eines Monosaccharides kann unverzweigt oder verzweigt sein. Unter "Oligosacchariden" werden Verbindungen verstanden, die durch Vereinigung von 2 bis 10 Monosaccharid-Moleküle unter Wasseraustritt erhalten werden und bei denen es sich um Glykoside oder Ether handelt. Im Zusammenhang mit der vorliegenden Erfindung werden unter "Polysacchariden" im Wesentlichen zwei Gruppen von Substanzen verstanden, nämlich einerseits Verbindungen, die als Gerüststoffe für Pflanzen und einige Tiere dienen, und andererseits Verbindungen, die als Reservestoffe für einfache Kohlenhydrate dienen und bei Bedarf im Organismus durch bestimmte Enzyme freigesetzt werden. Polysaccharide umfassen sowohl Homoglykane als auch Heteroglykane.

Im Zusammenhang mit der vorliegenden Erfindung wird unter einem "Kohlenhydrat-Gemisch" ein Gemisch aus zwei oder mehr chemisch verschiedenen Kohlenhydraten verstanden, das vorzugsweise keine anderen Bestandteile außer Kohlenhydraten enthält und das ohne vorherige chemische Reaktion gebildet wird. Bei dem zu oxidierenden Kohlenhydrat-Gemisch kann es sich sowohl um ein homogenes Gemisch als auch ein heterogenes Gemisch handeln. Ein heterogenes Gemisch besteht aus mindestens zwei Phasen, die die Kohlenhydrat-Einzelkomponenten enthalten oder die aus den Kohlenhydrat-Einzelkomponenten bestehen. Ein homogenes Gemisch ist dadurch gekennzeichnet, dass die Kohlenhydrat-Einzelkomponenten untereinander molekular dispers verteilt sind, ohne dass zwischen den Einzelkomponenten eine Grenzflächen besteht, wobei die Kohlenhydrat-Einzelkomponenten in beliebigen Mengenverhältnissen vorliegen können. Liegt das zu oxidierende Gemisch nicht in flüssiger Form vor beziehungsweise enthält das zu oxidierende Gemisch eine feste Phase, so wird das Gemisch erfindungsgemäß vor der selektiven Oxidation beispielsweise durch Herstellen einer wässrigen Lösung in die Flüssigphase überführt.

Eine Ausführungsform der Erfindung betrifft die Verwendung eines zu oxidierenden Kohlenhydrat-Gemisches als Ausgangssubstrat, bei dem mindestens ein Kohlenhydrat-Bestandteil ein C1-Kohlenstoffatom mit einer oxidierbaren Aldehyd-Gruppe enthält beziehungsweise bei dem vor der selektiven Oxidation das C1-Kohlenstoffatom mindestens eines Kohlenhydrat-Bestandteils mit einer oxidierbaren Aldehyd-Funktion versehen werden kann.

Eine bevorzugte Ausführungsform der Erfindung betrifft die Verwendung eines zu oxidierenden Kohlenhydrat-Gemisches als Ausgangssubstrat, bei dem entweder mehrere, besonders bevorzugt alle Kohlenhydrat-Bestandteile ein C1-Kohlenstoffatom mit einer oxidierbaren Aldehyd-Gruppe enthalten oder bei dem vor der Oxidation das C1-Kohlenstoffatom mehrerer, besonders bevorzugt aller Kohlenhydrat-Bestandteile mit einer oxidierbaren Aldehyd-Funktion versehen werden kann. Das zu oxidierende Kohlenhydrat-Gemisch enthält also vorzugsweise mehrere Kohlenhydrate, die entweder an ihrem C1-Kohlenstoff bereits eine oxidierbare Aldehyd-Gruppe aufweisen oder an deren C1-Kohlenstoffatom vor der selektiven Oxidation eine oxidierbare Aldehyd-Gruppe eingeführt werden kann. In dieser Ausführungsform kann das zu oxidierende Kohlenhydrat-Gemisch also mehrere unterschiedliche Aldosen und/oder 2-Ketosen umfassen. Besonders bevorzugt besteht das zu oxidierende Kohlenhydrat-Gemisch ausschließlich aus Kohlenhydrat-Einzelkomponenten, die entweder an ihrem C1-Kohlenstoff bereits eine oxidierbare Aldehyd-Gruppe aufweisen oder an deren C1-Kohlenstoffatom vor der Oxidation eine oxidierbare Aldehyd-Gruppe eingeführt werden kann. In dieser Ausführungsform besteht das zu oxidierende Kohlenhydrat-Gemisch also ausschließlich aus unterschiedlichen Aldosen und/oder 2-Ketosen.

Im Zusammenhang mit der vorliegenden Erfindung wird unter einer "mindestens ein Kohlenhydrat oder ein Kohlenhydrat-Gemisch enthaltenden Zusammensetzung" ein Gemisch unterschiedlicher chemischer Verbindungen verstanden, wobei mindestens ein Bestandteil der Zusammensetzung ein Kohlenhydrat ist, dessen C1-Kohlenstoffatom eine freie Aldehyd-Gruppe aufweist beziehungsweise vor der Oxidation mit einer oxidierbaren Aldehyd-Gruppe versehen werden kann. Bei den anderen Bestandteilen der zu oxidierenden Zusammensetzung kann es sich beispielsweise um andere, nicht-oxidierbare Kohlenhydrate, Eiweißstoffe, Pektine, Säuren, Fette, Salze, Aromastoffe wie Vanillin oder Furfurale, etc. oder Gemische davon handeln. Die zu oxidierende Zusammensetzung kann selbstverständlich mehrere unterschiedliche Aldosen und/oder 2-Ketosen umfassen.

Die vorliegende Erfindung betrifft daher die selektive Oxidation eines Kohlenhydrates, eines Gemisches unterschiedlicher Kohlenhydrate oder einer diese(s) enthaltenden Zusammensetzung unter Verwendung eines Metalloxid-geträgerten Gold-Katalysators, wobei es sich bei den Kohlenhydraten vorzugsweise um Monosaccharide oder Oligosaccharide handelt.

In bevorzugter Ausführungsform der Erfindung ist das zu oxidierende Monosaccharid eine Aldose wie Glucose, Galactose, Mannose, Xylose oder Ribose. Bei der Oxidation von Glucose wird.unter Verwendung des erfindungsgemäßen Verfahrens Gluconsäure als Oxidationsprodukt erhalten. Bei der Oxidation von Galactose wird unter Verwendung des erfindungsgemäßen Verfahrens Galactonsäure als Oxidationsprodukt erhalten.

In einer weiteren besonders bevorzugten Ausführungsform handelt es sich bei dem zu oxidierenden Kohlenhydrat um ein Oligosaccharid, insbesondere um ein Disaccharid. Das zu oxidierende Disaccharid ist vorzugsweise eine Disaccharid-Aldose wie Maltose, Lactose, Cellobiose oder Isomaltose. Erfindungsgemäß wird bei der selektiven Oxidation von Maltose unter Verwendung des erfindungsgemäßen Verfahrens Maltobionsäure als Oxidationsprodukt erhalten. Unter Verwendung des erfindungsgemäßen Verfahrens wird bei der Lactose-Oxidation Lactobionsäure als Oxidationsprodukt erhalten.

In einer weiteren besonders bevorzugten Ausführungsform der Erfindung wird als zu oxidierendes Oligosaccharid eine Disaccharid-Ketose eingesetzt. Bei der zu oxidierenden Disaccharid-Ketose handelt es sich vorzugsweise um Palatinose (Isomaltulose). Vor der Oxidation wird Palatinose erfindungsgemäß in die tautomere Aldose-Form überführt, die dann oxidiert wird.

In einer weiteren besonders bevorzugten Ausführungsform der Erfindung handelt es sich bei dem zu oxidierenden Kohlenhydrat um ein Maltodextrin. Maltodextrine sind durch den enzymatischen Stärkeabbau gewonnene wasserlösliche Kohlenhydrate, insbesondere Dextrose-Äquivalente, mit einer Kettenlänge von 2 bis 30, vorzugsweise 5 bis 20 Anhydroglucose-Einheiten und einem Anteil an Maltose. Bei der selektiven Oxidation von Maltodextrin wird unter Verwendung des erfindungsgemäßen Verfahrens ein Oxidationsprodukt erhalten, das erfindungsgemäß entsprechend der Zusammensetzung neben den Oligosaccharid-Aldonsäuren einen Anteil von Maltobionsäure und Gluconsäure aufweist.

In einer weiteren besonders bevorzugten Ausführungsform ist das zu oxidierende Kohlenhydrat-Gemisch beziehungsweise die zu oxidierende Kohlenhydrat-Zusammensetzung ein Stärkesirup. Unter einem Stärkesirup wird Glucosesirup verstanden, der aus Stärke gewonnen wird und als gereinigte wässrige Lösung vorliegt, wobei die Trockenmasse mindestens 70 % beträgt.

In einer weiteren Ausführungsform handelt es sich bei dem zu oxidierenden Kohlenhydrat um ein Furfural. Das zu oxidierende Furfural ist vorzugsweise Hydroxymethylfurfural (HMF) oder Glycosyloxymethylfurfural (GMF).

In bevorzugter Ausführungsform des erfindungsgemäßen Verfahrens zur selektiven Kohlenhydrat-Oxidation wird eine wässrige Lösung des zu oxidierenden Kohlenhydrates, Kohlenhydrat-Gemisches oder einer diese(s) enthaltenden Zusammensetzung hergestellt, die das Kohlenhydrat oder das Kohlenhydrat-Gemisch in einer Menge von mindestens etwa 10 mmol/l, bevorzugter mindestens etwa 100 mmol/l, 150 mmol/l, 200 mmol/l oder 250 mmol/l und am bevorzugtesten mindestens etwa 1000 mmol/l oder 1500 mmol/l enthält. Anschließend wird der wässrigen Kohlenhydrat-Lösung der vorzugsweise pulverförmige Metalloxid-geträgerte Gold-Katalysator in einer Menge von etwa 100 mg/l bis 10 g/l zugesetzt, wobei vorzugsweise pro Liter etwa 1 g Katalysator eingesetzt werden. Erfindungsgemäß beträgt das Verhältnis zwischen der Menge des/der zu oxidierenden Kohlenhydrate(s) oder des Kohlenhydrat-Gemisches und der Menge des auf dem Metalloxid-Trägers enthaltenen Goldes mindestens etwa 300, bevorzugter mehr als 350, 500, 1000, 1500, 2000, 2500, 3000, 3500 oder 4000 und am bevorzugtesten mehr als 9000, 10 000, 15 000, 20 000, 25 000, 30 000, 35 000 oder 40 000.

In bevorzugter Ausführungsform des erfindungsgemäßen Verfahrens zur selektiven Kohlenhydrat-Oxidation wird die selektive Oxidation des mindestens einen Kohlenhydrates, des Kohlenhydrat-Gemisches oder der diese(s) enthaltenden Zusammensetzung bei einem pH-Wert von 7 bis 11, vorzugsweise 8 bis 10, durchgeführt. Zur Durchführung der Kohlenhydrat-Oxidation wird erfindungsgemäß eine Temperatur von 20°C bis 140°C, vorzugsweise 40°C bis 90°C, besonders bevorzugt 50°C bis 80°C eingesetzt. Erfindungsgemäß beträgt der Druck etwa 1 bar bis etwa 25 bar. Während der Oxidation wird erfindungsgemäß Sauerstoff und/oder Luft mit einer Begasungsrate von 100 ml/ (mln x L_{Reaktorvolumen}) bis 10000 ml/ (min x L_{Reaktorvolumen}), vorzugsweise von 500 ml/(min x L_{Reaktorvolumen}), durch die wässrige Lösung des Kohlenhydrates, des Gemisches oder der Zusammensetzung hindurchgeperlt.

Erfindungsgemäß ist auch vorgesehen, dass zur selektiven Oxidation von Oligosacchariden ein Gold-Katalysator eingesetzt wird, der nanodispers verteilte Gold-Partikel auf einem Träger umfasst, wobei der Träger entweder ein Metalloxid-Träger ist.

Zur Herstellung der erfindungsgemäß zur Oligosaccharid-Oxidation verwendeten Metalloxid-geträgerten Gold-Katalysatoren können insbesondere Fällungsverfahren eingesetzt werden, wobei die Goldpartikel durch Fällung auf den Metalloxid-Träger abgeschieden oder gemeinsam mit einem Präcursor des Metalloxid-Trägers gefällt werden. Besonders geeignet sind Präzipitationsverfahren, Abscheidungs-Präzipitations-Verfahren und Verfahren zur chemische Abscheidung aus der Gasphase (CVD-Verfahren).

Erfindungsgemäß wird der zur selektiven Oligosaccharid-Oxidation verwendete Kohlenstoff-geträgerte Gold-Katalysator in Flüssigphase eingesetzt, wobei der Katalysator als Feststoff vorliegt, während die Oligosaccharide in Flüssigphase vorhanden sind. Der zur Oxidation eingesetzte Disauerstoff wird als Gas durch die Flüssigphase hindurchgeperlt und durch intensives Rühren in der Flüssigphase gelöst. Der Metalloxid-geträgerte Gold-Katalysator wird vorzugsweise in Form eines Pulvers oder Granulats eingesetzt.

Unter "Oligosacchariden" werden im Zusammenhang mit der vorliegenden Erfindung Verbindungen verstanden, die durch Vereinigung von 2 bis 10 Monosaccharid-Molekülen unter Wasseraustritt erhalten werden und bei denen es sich um Glykoside oder Ether handelt. Der Begriff "Oligosaccharid" umfasst auch Derivate, also Abkömmlinge eines Oligosaccharides, die aus einem Oligosaccharid in ein oder mehreren Reaktionsschritten gebildet werden. Erfindungsgemäß bevorzugt handelt es sich bei den zu oxidierenden Oligosacchariden insbesondere um Disaccharide, Trisaccharide etc.. Die zu oxidierenden Oligosaccharide enthalten an ihrem C1-Kohlenstoffatom eine oxidierbare Aldehyd-Gruppe, die durch die Oxidation in eine Carboxy-Gruppe überführt wird. Hingegen werden Alkohol-Gruppen der Oligosaccharide nicht oxidiert.

Bei den erfindungsgemäß eingesetzten Oligosacchariden kann es sich um Oligosaccharid-Aldosen handeln, die am C1-Kohlenstoff eine oxidierbare Aldehyd-Gruppe aufweisen, oder um Oligosaccharide in 2-Ketose-Form, bei denen am C1-Kohlenstoffatom eine oxidierbare Aldehyd-Funktion eingeführt werden kann.

Im Zusammenhang mit der vorliegenden Erfindung wird unter einer "Oligosaccharid-Aldose" ein Oligosaccharid verstanden, das am C1-Kohlenstoff eine Aldehyd-Gruppe (-CHO) besitzt. Durch die selektive Oxidation der Aldehyd-Gruppe einer Oligosaccharid-Aldose wird eine Oligosaccharid-Aldonsäure erhalten. Unter einer "Oligosaccharid-Aldonsäure" wird eine Oligosaccharidsäure verstanden, die durch Oxidation einer Aldehyd-Gruppe eines Oligosaccharides zur Carboxy-Gruppe erhalten wird.

Die vorliegende Erfindung betrifft daher auch besonders ein Verfahren zur Herstellung einer Oligosaccharid-Aldonsäure oder eines Gemisches verschiedener Oligosaccharid-Aldonsäuren durch selektive Oxidation einer oder mehrerer Oligosaccharid-Aldosen mit einer oxidierbaren Aldehyd-Gruppe unter Verwendung eines Metalloxid-geträgerten Gold-Katalysators.

Unter einer "2-Ketose" wird ein Oligosaccharid verstanden, dass eine in 2-Stellung befindliche Keto-Gruppe besitzt. In 2-Ketose-Form vorliegende Oligosaccharide können in Oligosaccharid-Aldosen überführt werden, wobei die 2-Ketose zunächst in die Enol-Form überführt wird, die dann zu der oxidierbaren Oligosaccharid-Aldose tautomerisiert wird.

Die vorliegende Erfindung betrifft daher auch besonders ein Verfahren zur Herstellung einer Oligosaccharid-Aldonsäure oder eines Gemisches verschiedener Oligosaccharid-Aldonsäuren unter Verwendung einer oder mehrerer Oligosaccharide in 2-Ketose-Form, wobei die 2-Ketose(n) zunächst in die tautomere(n) Oligosaccharid-Aldose-Form(en) mit einer oxidierbaren Aldehyd-Gruppe überführt werden, die dann unter Verwendung eines Metalloxid-geträgerten Gold-Katalysators selektiv oxidiert wird/werden.

Im Zusammenhang mit der vorliegenden Erfindung wird unter einem "Oligosaccharid-Gemisch" ein Gemisch aus zwei oder mehr chemisch verschiedenen Oligosacchariden verstanden. Das zu oxidierende Oligosaccharid-Gemisch enthält in bevorzugter Ausführungs außer Oligosacchariden keine anderen Bestandteile. Bei dem zu oxidierenden Oligosaccharid-Gemisch kann es sich sowohl um ein homogenes Gemisch als auch ein heterogenes Gemisch handeln. Liegt das zu oxidierende Oligosaccharid-Gemisch nicht in flüssiger Form vor beziehungsweise enthält das zu oxidierende Oligosaccharid-Gemisch eine feste Phase, so wird das Gemisch vor der selektiven Oxidation in die Flüssigphase überführt, beispielsweise durch Herstellen einer wässrigen Lösung.

Eine Ausführungsform der Erfindung betrifft die Verwendung eines zu oxidierenden Oligosaccharid-Gemisches als Ausgangssubstrat, bei dem mindestens ein Oligosaccharid-Bestandteil ein C1-Kohlenstoffatom mit einer oxidierbaren Aldehyd-Gruppe enthält beziehungsweise bei dem vor der selektiven Oxidation das Cl-Kohlenstoffatom mindestens eines Oligosaccharid-Bestandteils mit einer oxidierbaren Aldehyd-Funktion versehen werden kann.

Eine weitere bevorzugte Ausführungsform der Erfindung betrifft die Verwendung eines zu oxidierenden Oligosaccharid-Gemisches als Ausgangssubstrat, wobei das zu oxidierende Gemisch vorzugsweise mehrere Oligosaccharide enthält, die entweder an ihrem C1-Kohlenstoff bereits eine oxidierbare Aldehyd-Gruppe aufweisen oder an deren Cl-Kohlenstoffatom vor der selektiven Oxidation eine oxidierbare Aldehyd-Gruppe eingeführt werden kann. In dieser Ausführungsform kann das zu oxidierende Oligosaccharid also mehrere unterschiedliche Oligosaccharid-Aldosen und/oder Oligosaccharide in 2-Ketose-Form umfassen. Besonders bevorzugt besteht das zu oxidierende Oligosaccharid-Gemisch ausschließlich aus Oligosaccharid-Einzelkomponenten, die entweder an ihrem C1-Kohlenstoff bereits eine oxidierbare Aldehyd-Gruppe aufweisen oder an deren C1-Kohlenstoffatom vor der selektiven Oxidation eine oxidierbare Aldehyd-Gruppe eingeführt werden kann. In dieser Ausführungsform besteht das zu oxidierende Oligosaccharid also ausschließlich aus unterschiedlichen Oligosaccharid-Aldosen und/oder Oligosacchariden in 2-Ketose-Form.

Im Zusammenhang mit der vorliegenden Erfindung wird unter einer "mindestens ein Oligosaccharid oder ein Oligosaccharid-Gemisch enthaltenden Zusammensetzung" ein Gemisch unterschiedlicher chemischer Verbindungen verstanden, wobei mindestens ein Bestandteil der Zusammensetzung ein Oligosaccharid ist, dessen C1-Kohlenstoffatom eine freie Aldehyd-Gruppe aufweist beziehungsweise vor der selektiven Oxidation mit einer oxidierbaren Aldehyd-Gruppe versehen werden kann. Bei den anderen Bestandteilen der zu oxidierenden Zusammensetzung kann es sich beispielsweise um andere Kohlenhydrate, beispielsweise nicht-oxidierbare oder selektiv oxidierbare Monosaccharide, Eiweißstoffe, Pektine, Säuren, Fette, Salze, Aromastoffe etc. handeln. Die zu oxidierende Zusammensetzung kann selbstverständlich mehrere unterschiedliche Oligosaccharid-Aldosen und/oder Oligosaccharide in 2-Ketose-Form enthalten.

In einer besonders bevorzugten Ausführungsform handelt es sich bei dem zu oxidierenden Oligosaccharid um ein Disaccharid. Das zu oxidierende Disaccharid kann eine Disaccharid-Aldose wie Maltose, Lactose, Cellobiose oder Isomaltose sein. Erfindungsgemäß wird bei der selektiven Oxidation von Maltose unter Verwendung des erfindungsgemäßen Verfahrens Maltobionsäure als Oxidationsprodukt erhalten. Unter Verwendung des erfindungsgemäßen Verfahrens wird bei der Lactose-Oxidation Lactobionsäure als Oxidationsprodukt erhalten.

In einer weiteren besonders bevorzugten Ausführungsform der Erfindung wird als zu oxidierendes Oligosaccharid eine Disaccharid-Ketose eingesetzt. Bei der zu oxidierenden Disaccharid-Ketose handelt es sich vorzugsweise um Palatinose. Vor der Oxidation wird Palatinose erfindungsgemäß in die tautomere Aldose-Form überführt, die dann oxidiert wird.

In einer weiteren besonders bevorzugten Ausführungsform der Erfindung handelt es sich bei dem zu oxidierenden Oligosaccharid-Gemisch beziehungsweise der zu oxidierenden Oligosaccharid-haltigen Zusammensetzung um ein Maltodextrin. Bei der selektiven Oxidation von Maltodextrin wird unter Verwendung des erfindungsgemäßen Verfahrens ein Oxidationsprodukt erhalten, das erfindungsgemäß einen Anteil von Maltobionsäure und Gluconsäure aufweist.

In einer weiteren besonders bevorzugten Ausführungsform ist die zu oxidierende Oligosaccharidhaltige Zusammensetzung ein Stärkesirup.

Je nach verwendetem Ausgangsmaterial kann es sich bei den erfindungsgemäß hergestellten Oxidationsprodukten entweder um eine Aldonsäure, ein Gemisch unterschiedlicher Aldonsäuren oder eine Zusammensetzung handeln, die neben Bestandteilen wie Fetten, Pektinen, Eiweißstoffen, Salzen, Aromastoffen etc. ein oder mehrere Aldonsäuren umfasst. Insbesondere wenn als Edukt ein einziges Kohlenhydrat eingesetzt wird, weisen die erfindungsgemäß erhaltenen Oxidationsprodukte aufgrund der außerordentlich hohen Selektivität der erfindungsgemäß verwendeten Gold-Katalysatoren eine sehr hohe Reinheit auf. Die erfindungsgemäß erhaltenen Oxidationsprodukte zeichnen sich gegenüber herkömmlichen Produkten auch dadurch aus, dass sie frei von möglicherweise gesundheitsschädlichen mikrobiellen Stoffwechselprodukten sind.

Gluconsäure, die durch selektive Oxidation von Glucose erhältlich ist, zeichnet sich durch eine hohe Reinheit aus, wobei der Anteil von Gluconsäure am Gesamtoxidationsprodukt mehr als 95%, vorzugsweise mehr als 97%, bevorzugter mehr als 98% und am bevorzugtesten mehr als 99% beträgt.

Die unter Verwendung der erfindungsgemäßen Verfahren herstellbare Gluconsäure kann, gegebenenfalls nach weiteren Aufreinigungsschritten unter Verwendung geeigneter Verfahren wie chromatographischer Verfahren, als Zusatz zu Lebensmitteln, Getränken oder Tierfutter, für kosmetische Anwendungen, pharmazeutische Anwendungen oder als Detergens, beispielsweise in Reinigungsmitteln, eingesetzt werden. Gluconsäure besitzt beispielsweise hervorragende Eigenschaften als Antioxidationsmittel. Ferner ist bekannt, dass Gluconsäure ein hervorragendes Mittel zur Hautpflege darstellt.

Maltobionsäure, die durch selektive Oxidation von Maltose erhältlich ist, zeichnet sich durch eine hohe Reinheit aus, wobei der Anteil von Maltobionsäure am Gesamtoxidationsprodukt mehr als 95%, vorzugsweise mehr als 97%, bevorzugter mehr als 98% und am bevorzugtesten mehr als 99% beträgt.

Die unter Verwendung der erfindungsgemäßen Verfahren herstellbare Maltobionsäure kann, gegebenenfalls nach weiteren Aufreinigungsschritten, als Zusatz zu Lebensmitteln, Getränken oder Tierfutter oder für pharmazeutische Anwendungen eingesetzt werden. Maltobionsäure kann beispielsweise nach Aufreinigung mittels Ionenaustausch-Chromatographie in die Lacton-Form überführt und dann beispielsweise mit 1,3-Diamino-2-propanal kondensiert werden, wobei das Bisamid von Maltobionsäure erhalten wird. Das Bisamid von Maltobionsäure kann als oral applizierbares Antikoagulant und/oder als Antithrombolytikum eingesetzt werden.

Lactobionsäure, die durch Oxidation von Lactose erhältlich ist, zeichnet sich durch eine hohe Reinheit aus, wobei der Anteil von Lactobionsäure am Gesamtoxidationsprodukt mehr als 95%, vorzugsweise mehr als 97, bevorzugter mehr als 98% und am bevorzugtesten mehr als 99% beträgt.

Die unter Verwendung der erfindungsgemäßen Verfahren herstellbare Lactobionsäure kann, gegebenenfalls nach weiteren Aufreinigungsschritten, als Zusatz zu Lebensmitteln, Getränken oder Tierfutter und für kosmetische Anwendungen sowie pharmazeutische Anwendungen eingesetzt werden. Aufgrund ihrer hygroskopischen Eigenschaften kann Lactobionsäure atmosphärisches Wasser binden, so dass Lactobionsäure eine natürliche Gelmatrix bildet. Die so gebildete Gelmatrix enthält etwa 14% Wasser. Aufgrund ihrer Fähigkeit, eine solche Gelmatrix zu bilden, stellt Lactobionsäure wie Gluconsäure ein hervorragendes Mittel zur Hautpflege dar. Lactobionsäure weist ferner hervorragende Eigenschaften zur Metallchelat-Bildung auf und lässt sich daher zur Herstellung einer Transplantationslösung, beispielsweise der Wisconsin-Transplantationslösung, einsetzen, die zum Transport und zur Aufbewahrung von zu transplantierenden Organen dient. Lactobionsäure lässt sich auch als Cobuilder in Waschpulver einsetzen, wobei das Waschpulver bis zu 40% Lactobionsäure enthalten kann. Da Lactobionsäure einen milden süß-sauren Geschmack aufweist, kann sie ebenfalls zur Herstellung von Lebensmitteln und Tierfutter eingesetzt werden.

Die vorliegende Erfindung betrifft daher die Ver Als Oxidationsprodukt kann ein Produktgemisch erhalten werden, das einen hohen Anteil von Gluconsäure und Maltobionsäure enthält. Dieses ist und durch Oxidation von Maltodextrin erhältlich.

Ein durch selektive Maltodextrin-Oxidation erhaltene Maltobionsäure- und Gluconsäure-haltige Oxidationsprodukt kann, gegebenenfalls nach weiteren Aufreinigungsschritten, insbesondere zur weiteren Anreicherung von Maltobionsäure und Gluconsäure, insbesondere zur Herstellung von Lebensmitteln und pharmazeutischen Zusammensetzungen eingesetzt werden.

Als Oxidationsprodukt kann ein Produkt erhalten werden, das einen hohen Anteil an Maltobionsäure aufweist. Dieses ist durch selektive Oxidation eines Stärkesirups erhältlich. Das bei der Oxidation von Stärkesirup erhaltene Oxidationsprodukt kann insbesondere zur Herstellung von Lebensmitteln und Tierfutter eingesetzt werden.

Die vorliegende Erfindung betrifft ebenfalls die Verwendung eines Gold-Katalysators, umfassend nanodispers verteilte Gold-Partikel auf einem Metalloxid-Träger, zur selektiven Oxidation von mindestens einem Kohlenhydrat, einem Kohlenhydrat-Gemisch oder eine diese(s) enthaltenden Zusammensetzung. Bei dem zur selektiven Oxidation von einem Kohlenhydrat, dem Gemisch oder einer diese enthaltende Zusammensetzung handelt es sich erfindungsgemäß bevorzugt um einen Gold-Katalysator, dessen Träger ein TiO₂-Träger ist. Der erfindungsgemäß verwendete Gold-Katalysator mit dem TiO₂-Träge enthält etwa 0,1 % bis 5 % Gold, vorzugsweise etwa 0,5 % bis 1 % Gold, besonders bevorzugt 0,5 % Gold. In einer weiteren bevorzugten Ausführungsform umfasst der erfindungsgemäß verwendete Gold-Katalysator einen Al₂O₃-Träger. Der erfindungsgemäß verwendete Gold-Katalysator mit dem Al₂O₃-Träger enthält etwa 0,1 % bis 5 % Gold, vorzugsweise etwa 0,5 % bis 1 % Gold. Bei dem zu oxidierenden Kohlenhydrat kann es sich sowohl um eine Kohlenhydrat-Aldose als auch um ein Kohlenhydrat in 2-Ketose-Form handeln. Das zu oxidierende Kohlenhydrat kann ein Monosaccharid, ein Oligosaccharid, ein Gemisch davon oder eine diese(s) enthaltenden Zusammensetzung sein. Wenn das zu oxidierende Monosaccharid Glucose ist, wird unter Verwendung des Gold-Katalysators als Glucose-Oxidationsprodukt Gluconsäure erhalten. Wenn das zu oxidierende Oligosaccharid eine Disaccharid-Aldose wie Maltose ist, wird als Oxidationsprodukt Maltobionsäure erhalten. Wenn das zu oxidierende Oligosaccharid eine Disaccharid-Aldose wie Lactose ist, wird als Oxidationsprodukt Lactobionsäure erhalten. In weiteren Ausführungsformen kann der erfindungsgemäß eingesetzte Gold-Katalysator zur Oxidation von Maltodextrin oder zu Oxidation eines Stärkesirups eingesetzt werden.

Die vorliegende Erfindung betrifft daher auch die Verwendung eines Metalloxid-geträgerten Gold-Katalysators zur Herstellung einer oder mehrerer Aldonsäure(n) aus einer oder mehreren Kohlenhydrat-Aldose(n). Die vorliegende Erfindung betrifft auch die Verwendung eines Metalloxid-geträgerten Gold-Katalysators zur Herstellung einer oder mehrerer Aldonsäure(n) aus einem oder mehreren Kohlenhydrat(en) in 2-Ketose-Form, wobei das/die Kohlenhydrat(e) in 2-Ketose-Form vor der Oxidation zunächst in die tautomere(n) Aldose-Form(en) überführt und dann selektiv oxidiert wird/werden.

Die vorliegende Erfindung wird anhand der folgenden Figuren und Beispiele näher erläutert. Die Figuren zeigen:
Figur 1 zeigt mittels eines UV-Detektors erhaltene Chromatogramme der Produktgemische, die bei der katalytischen Oxidation von Glucose unter Verwendung von Platin- und Palladium-Katalysatoren erhalten wurden. A: Pt-Bi/C-Katalysator; B: Pt/Al₂O₃-Katalysator.
Figur 2 zeigt mittels eines UV-Detektors erhaltene Chromatogramme der Produkte, die erfindungsgemäß bei der katalytischen Oxidation von Glucose unter Verwendung von Gold-Katalysatoren erhalten wurden. A: 0,95% Au/Al₂O₃-Katalysator; B: 0,5% Au/TiO₂-Katalysator.
Figur 3 zeigt mittels eines UV-Detektors erhaltene Chromatogramme der Produkte, die bei der katalytischen Oxidation von Lactose unter Verwendung von Platin-, Palladium- und Gold-Katalysatoren erhalten wurden. A: Pt/Al₂O₃-Katalysator; B: Pd/Al₂O₃-Katalysator; C: 0,5% Au/TiO₂-Katalysator.
Figur 4 zeigt mittels eines UV-Detektors erhaltene Chromatogramme der Produkte, die bei der katalytischen Oxidation von Maltose unter Verwendung von Platin-, Palladium- und Gold-Katalysatoren erhalten wurden. A: Pt/Al₂O₃-Katalysator; B: Pd/Al₂O₃-Katalysator; C: 0,5% Au/TiO₂-Katalysator.
Figur 5 zeigt mittels eines UV-Detektors erhaltene Chromatogramme der Produkte, die bei der katalytischen Oxidation von Maltodextrin unter Verwendung von Platin-, Palladium- und Gold-Katalysatoren erhalten wurden. A: Pt/Al₂O₃-Katalysator; B: Pd/Al₂O₃-Katalysator; C: 0,5% Au/TiO₂-Katalysator

### Referenzbeispiel

### Glucose-Oxidation mit Platin- und Palladium-Katalysatoren

Als Katalysatoren wurden ein 5 % Platin- und 5 % Bismut-enthaltender Kohlenstoff-geträgerter Katalysator (Fa. Degussa), ein 5 % Platin-enthaltender Katalysator auf einem Al₂O₃-Träger (Fa. Engelhard) sowie ein 5 % Palladium-enthaltender Katalysator auf einem Al₂O₃-Träger eingesetzt. Die Glucoseoxidation erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 100 mmol/l |
| pH-Wert: | 11 |
| Temperatur: | 40°C |
| Druck: | 1 bar |
| O₂-Begasungsrate | 500 ml/min |
| Rührgeschwindigkeit: | 700 UpM |

Die Ergebnisse, die unter Verwendung der vorstehend aufgeführten Katalysatoren bei der Glucoseoxidation erhalten wurden, sind in der folgenden Tabelle 1 dargestellt.

**Tabelle 1: Vergleich der Pt- und Pd-Katalysatoren bezüglich Glucose-Oxidation, Anfangsaktivität bis 10 % Umsatz und Bildung von Fructose durch Isomerisierung**

| **Eigenschaft** | **Pt(5%)** | **5 %** | **5 %** |
|---|---|---|---|
| | **Bi (5%) /C** | **Pt/Al₂O₃** | **Pd/Al₂O₃** |
| Glucoseumsatz in %* | >95 | >95 | 90 |
| Anfangsaktivität in | 19 | 62 | 76 |
| mmol_{Gluco}- | | | |
| ₛₑ/ (g_{metal·}min) | | | |
| Selektivität zu | 85 | 83 | 92 |
| Gluconsäure in % | | | |
| Selektivität zu an- | 11 | 12 | 4 |
| deren Oxidations- | | | |
| produkten in % ** | | | |
| Selektivität zu | 2,5 | 2,5 | <0,5 |
| Fructose in % | | | |

| | | | |
|---|---|---|---|
| * Umsatz, bei dem die Reaktion abgebrochen wurde ** andere Oxidationsprodukte: Glucuronsäure, 2-und 5-Keto-Gluconsäure sowie Glucarsäure | | | |

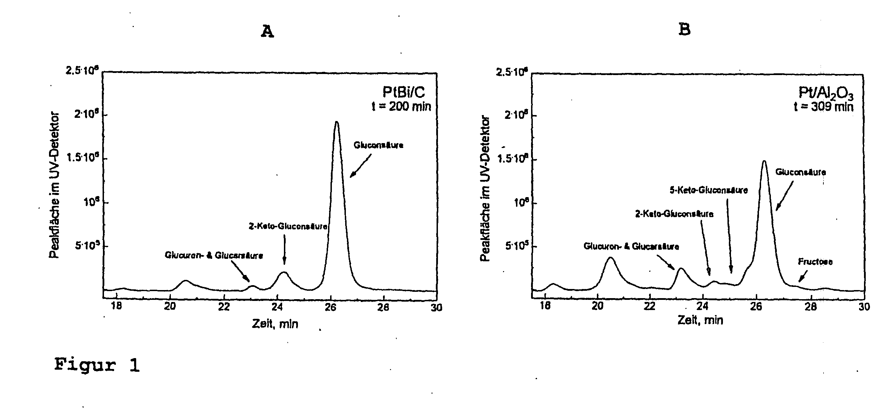

Tabelle 1 zeigt, dass herkömmliche Platin- und Palladium-Katalysatoren bei der Glucose-Oxidation nur eine mäßige Selektivität bezüglich der Bildung von Gluconsäure aufweisen und dass erhebliche Mengen an anderen Oxidationsprodukten wie Glucoronsäure, Glucarsäure, 2-Keto-Gluconsäure und 5-Keto-Gluconsäure gebildet werden. Dieses Produktspektrum ist auch in Figur 1 zu erkennen, die mittels eines UV-Detektors erhaltene Produkt-Chromatogramme zeigt. Aus den erhaltenen Ergebnissen geht hervor, dass die herkömmlicherweise verwendeten Platin- oder Palladium-Katalysatoren für die selektive Herstellung von Gluconsäure durch Glucose-Oxidation nicht geeignet sind.

### Beispiel 1:

### Glucose-Oxidation unter Verwendung von Gold-Katalysatoren

Für dieses Beispiel wurden die folgenden Katalysator-Typen eingesetzt:
- A:: 1 % Au/C Typ 138 (ACA) (Vergleich)
- B:: 0,95 % Au/Al₂O₃ (ACA) (erfindungsgemäß)
- C:: 0,7 % Au/C Typ 11 (ACA) (Vergleich)
- D:: 1 % Au/TiO₂ Typ 5 (ACA) (erfindungsgemäß)
- E:: 0,5 % Au/TiO₂ Typ 102 (ACA) (erfindungsgemäß)
- F:: 0,5 % Au/TiO₂ Typ 149 (ACA) (erfindungsgemäß)

Die Glucose-Oxidation erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 100 mmol/l |
| pH-Wert: | 11 |
| Temperatur: | 40°C |
| Druck: | 1 bar |
| O₂-Begasungsrate: | 500 ml/min |
| Rührgeschwindigkeit: | 700 UpM |

Die Ergebnisse, die bei der Glucose-Oxidation unter Verwendung der vorstehend aufgeführten Katalysatoren erhalten wurden, sind in Tabelle 2 dargestellt.

**Tabelle 2: Vergleich verschiedener Gold-Katalysatoren bezüglich Selektivität der Glucose-Oxidation, Anfangsaktivität bis 10 % Umsatz und Fructose-Bildung infolge Isomerisierung**

| **Eigenschaft** | **A** | **B** | **C** | **D** | **E** | **F** |
|---|---|---|---|---|---|---|
| | | | | | | |
| Glucoseumsatz in | 93 | 97 | 88 | 74 | 95 | 100 |
| %* | | | | | | |
| | | | | | | |
| Anfangsaktivität | 156 | 133 | 390 | 105 | 371 | 550 |
| in mmol_{Gluco-} | | | | | | |
| ₛₑ/g_{Metall ·} min) | | | | | | |
| | | | | | | |
| Selektivität zu | 95 | 95 | 97 | 95 | 98 | 98 |
| Gluconsäure in % | | | | | | |
| | | | | | | |
| Selektivität zu | 0 | <2 | <1 | 0 | 0 | <1 |
| anderen Oxidati- | | | | | | |
| onsprodukten- | | | | | | |
| Produkten in %** | | | | | | |
| | | | | | | |
| Selektivität zu | 5 | 3 | 2 | 5 | 1 | 1 |
| Fructose in % | | | | | | |

| | | | | | | |
|---|---|---|---|---|---|---|
| * Umsatz, bei dem die Reaktion abgebrochen wurde ** andere Oxidationsprodukte: Glucuronsäure, 2- und 5-Keto-Gluconsäure, Glucarsäure | | | | | | |

Aus Tabelle 2 geht hervor, dass die eingesetzten Gold-Katalysatoren eine Aktivität bezüglich der Glucose-Oxidation aufweisen, die gegenüber der Aktivität der in dem Vergleichsbeispiel verwendeten Platin- und Palladium-Katalysatoren um mindestens eine Größenordnung oder noch mehr höher ist.

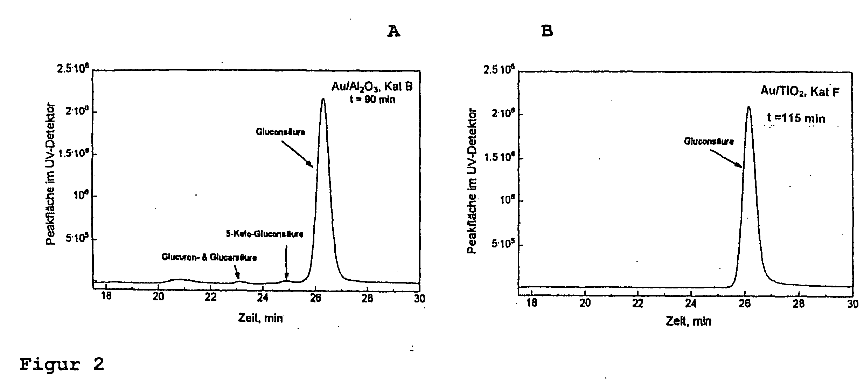

Tabelle 2 zeigt darüber hinaus die außerordentlich hohe Selektivität der verwendeten Gold-Katalysatoren für die Bildung von Gluconsäure aus Glucose. Die hohe Selektivität der eingesetzten Gold-Katalysatoren bezüglich der Gluconsäure-Herstellung ist auch in Figur 2 zu erkennen. Bei Verwendung des Au/TiO₂-Katalysatores (Katalysator F) (Figur 2B) wird nur Gluconsäure gebildet, während andere Oxidationsprodukte nicht nachweisbar sind. Hingegen werden bei Verwendung des Au/Al₂O₃-Katalysators (Katalysator B) neben Gluconsäure auch geringe Mengen anderer Oxidationsprodukten wie Glucuronsäure, Glucarsäure und 5-Keto-Gluconsäure gebildet. Die Menge dieser Oxidationsprodukte ist jedoch im Vergleich zu den Mengen dieser Oxidationsprodukte, die bei Verwendung von Platin- oder Palladium-Katalysatoren erhalten werden, deutlich geringer.

Bei den erfindungsgemäß eingesetzten Gold-Katalysatoren wurde auch festgestellt, dass diese nur zu einer sehr geringen Isomerisierung von Fructose führen, wobei die Fructose selbst aber nicht weiter oxidiert wird.

Anhand der erhaltenen Ergebnisse zeigt sich, dass die erfindungsgemäß eingesetzten Gold-Katalysatoren erheblich besser zur Herstellung von Gluconsäure aus Glucose geeignet sind als die im Vergleichsbeispiel verwendeten Platin- oder Palladium-basierten Katalysatoren.

### Beispiel 2:

### Glucose-Oxidation mit Au/TiO₂-Katalysatoren

Der in Beispiel 1 dargestellte 0,5 % Au-enthaltende Katalysator mit TiO₂-Träger (Katalysator G) wurde für weitergehende Untersuchungen ausgewählt, wobei die Glucose-Oxidation unter variierenden Reaktionsbedingungen getestet wurde.

Sofern nicht anders angegeben, wurden die gleichen Reaktionsbedingungen eingesetzt, wie in Beispiel 1 angeführt.

### Einfluss des pH-Wertes auf die Glucose-Oxidation

Die Ergebnisse der Glucose-Oxidation unter Verwendung des 0,5 % Au/TiO₂-Katalysators (Katalysator G) bei unterschiedlichen pH-Werten sind in Tabelle 3 dargestellt.

**Tabelle 3: Glucose-Oxidation mit einem 0,5% Au//TiO₂-Katalysator bei unterschiedlichen pH-Werten, Anfangsaktivität bis 10% Umsatz, Bildung von Fructose infolge Isomerisierung**

| **Eigenschaft** | **PH 7** | **pH 9** | **pH 11** |
|---|---|---|---|
| Glucoseumsatz in | 80 | 100 | 100 |
| %* | | | |
| Anfangsaktivität | 194 | 416 | 550 |
| in mmol_{Gluco-} | | | |
| se/(g_{Metall}·min) | | | |
| Selektivität zu | >99 | >99,5 | 98 |
| Gluconsäure in % | | | |
| Selektivität zu | <0,5 | 0 | <0,5 |
| anderen Ox. - | | | |
| produkten in %** | | | |
| Selektivität-zu | 0 | 0 | 1 |
| Fructose in % | | | |

| | | | |
|---|---|---|---|
| * Umsatz, bei dem die Reaktion abgebrochen wurde ** andere Oxidationsprodukte: Glucuronsäure, 2-und 5-Keto-Gluconsäure, Glucarsäure | | | |

Aus Tabelle 3 geht hervor, dass die Aktivität des Au/TiO₂-Katalysators im nur schwach alkalischen bis neutralen pH-Bereich etwas geringer ist, während die Selektivität noch zunimmt.

### Einfluss der Temperatur auf die Glucose-Oxidation

Die Ergebnisse der Glucose-Oxidation unter Verwendung des Au/TiO₂-Katalysators bei unterschiedlichen Temperaturen, einem pH-Wert von 9 und einer Glucose-Anfangskonzentration von 250 mmol/l sind in Tabelle 4 dargestellt.

**Tabelle 4: Glucose-Oxidation mit einem 0,5% Au//TiO₂₋ Katalysator bei unterschiedlichen Temperaturen, einer Glucose-Anfangskonzentration von 250 mmol/l und einem pH-Wert von 9, Anfangsaktivität bis 10% Umsatz, Bildung von Fructose infolge Isomerisierung**

| **Eigenschaft** | **40°C** | **50°C** | **70°C** |
|---|---|---|---|
| Glucoseumsatz in %* | 95 | 100 | 100 |
| Anfangsaktivität in | 320 | 1056 | 1404 |
| mmol_{Glucose}/ (g_{Metall·}min) | | | |
| Selektivität zu Glu- | >99,5 | >99 | 95,5 |
| consäure in %. | | | |
| Selektivität zu ande- | <0,1 | <0,1 | <0,2 |
| ren Ox.-produkten in | | | |
| %** | | | |
| Selektivität zu Fruc- | 0 | <0,5 | 3 |
| tose in % | | | |

| | | | |
|---|---|---|---|
| * Umsatz, bei dem die Reaktion abgebrochen wurde ** andere Oxidationsprodukte: Glucuronsäure, 2- & 5-Keto-Gluconsäure, Glucarsäure | | | |

Aus Tabelle 4 geht hervor, dass die Selektivität der Glucose-Oxidation mit steigender Reaktionstemperatur etwas abnimmt. Dies ist jedoch nicht auf die Bildung anderer Oxidationsprodukte als Gluconsäure zurückzuführen, sondern nahezu ausschließlich auf die vermehrte Bildung von Fructose entsprechend einer höheren Isomerisierungsgeschwindigkeit von Glucose zu Fructose.

### Einfluss der Glucose-Anfangskonzentration auf die Glucose-Oxidation

Der Einfluss der Glucose-Anfangskonzentration auf die Glucose-Oxidation unter Verwendung des 0,5% Au/TiO₂-Katalysators bei einer Temperatur von 40°C und einem pH-Wert von 9 ist in Tabelle 5 dargestellt.

**Tabelle 5: Glucose-Oxidation mit einem 0,5% Au//TiO₂-Katalysator bei unterschiedlichen Glucose-Anfangskonzentrationen und einem pH-Wert von 9, Anfangsaktivität bis 10% Umsatz, Bildung von Fructose infolge Isomerisierung**

| **Eigenschaft** | **10 mmol/l** | **250 mmol/l** | **1100 mmol/l** |
|---|---|---|---|
| Glucoseumsatz in | 40 | 95 | 100 |
| %* | | | |
| Anfangsaktivität | 20 | 320 | 1200 |
| in mmol_{Gluco-} | | | |
| ₛₑ/(g_{Metall}·min) | | | |
| Selektivität zu | 99,4 | 99,6 | 99,2 |
| Gluconsäure in % | | | |
| Selektivität zu | 0 | 0 | 0 |
| anderen Ox.- | | | |
| produkten in %** | | | |
| Selektivität zu | 0,6 | <0,1 | 0,4 |
| Fructose in % | | | |

| | | | |
|---|---|---|---|
| * Umsatz, bei dem die Reaktion abgebrochen wurde ** andere Oxidationsprodukte: Glucuronsäure, 2- & 5-Keto-Gluconsäure, Glucarsäure | | | |

Aus den in Tabelle 5 dargestellten Ergebnissen ist ersichtlich, dass der verwendete Goldkatalysator auch in konzentrierten Glucose-Lösungen (1100 mmol/l; etwa 20 % Glucose/l) eine ausgezeichnete Aktivität und Selektivität zeigt, wobei deutlich mehr Glucose in gleicher Reaktionszeit umgesetzt wird.

### Beispiel 3:

### Langzeitstabilität des Au/TiO₂-Katalysators bei der Glucoseoxidation

Die Langzeitstabilität des 0,5% Au/TiO₂-Katalysators (Kat G in Beispiel 1) wurde in Form von repeated batch-Versuchen getestet. Dazu wurde tagsüber eine Glucose-Oxidation durchgeführt. Über Nacht beziehungsweise übers Wochenende wurde der Katalysator in der Reaktionslösung, die hauptsächlich aus einer wässrigen Gluconsäure-Lösung besteht, belassen, so dass der Katalysator sedimentieren konnte. Am nächsten Tag wurde der Überstand abdekantiert und der Reaktor wurde mit frischer Glucose-Lösung aufgefüllt und die nächste Glucose-Oxidation wurde durchgeführt. Insgesamt wurden 17 Batch-Versuche bei einem pH-Wert von 9, einer Temperatur von 40°C und einer Glucose-Anfangskonzentration von 250 mmol/l (entspricht etwa 4,5 % Glucose/l) durchgeführt. Die weiteren Reaktionsbedingungen waren wie in Beispiel 1 beschrieben. Die Ergebnisse für einige ausgewählte Batch-Versuche sind in Tabelle 6 aufgeführt.

**Tabelle 6: Langzeitversuch zur Glucose-Oxidation mit einem 0,5% Au//TiO₂-Katalysat bei einer Glucose-Anfangskonzentration von 250 mmol/l, einer Temperatur von 40°C und einem pH-Wert von 9, Anfangsaktivität bis 10% Umsatz, Bildung von Fructose infolge Isomerisierung**

| **Eigenschaft** | **Batch-Nummer** | | | | |
|---|---|---|---|---|---|
| | **1** | **5** | **10** | **15** | **17** |
| Glucoseumsatz in | 95 | 97 | 99 | 94 | 56 |
| %** | | | | | |
| Anfangsaktivität | 320 | 350 | 460 | 430 | 570 |
| in mmol_{Gluco-} | | | | | |
| ₛₑ/ (g_{Metall}·min) | | | | | |
| Selektivität zu | 99,5 | 99,7 | 99,6 | 99 | 99,3 |
| Gluconsäure in % | | | | | |
| Selektivität zu | <0,1 | <0,1 | <0,1 | <0,1 | <0,1 |
| anderen Ox.- | | | | | |
| produkten in %** | | | | | |
| Selektivität zu | <0,1 | <0,1 | 0,1 | 0,3 | <0,3 |
| Fructose in % | | | | | |

| | | | | | |
|---|---|---|---|---|---|
| * Umsatz, bei dem die Reaktion abgebrochen wurde ** andere Oxidationsprodukte: Glucuronsäure, 2- & 5-Keto-Gluconsäure, Glucarsäure | | | | | |

Die in Tabelle 6 dargestellten Ergebnisse zeigen, dass die katalytischen Eigenschaften des Gold-Katalysators über den untersuchten Versuchszeitraum hinweg als konstant anzusehen sind und somit der Katalysator über eine äußerst hohe Lebensdauer verfügt. Insgesamt wurden in diesen Versuchen mit 0,5 g des 0,5 % Au/TiO₂-Katalysators etwa 360 g Glucose nahezu 100%-ig zu Gluconsäure umgesetzt. Dies entspricht etwa 145 t Glucose/kg Gold. Aufgrund des in dieser Rechnung noch nicht berücksichtigten Katalysatorverlustes, der während der Versuche durch Probenentnahme entsteht, liegt dieser Wert wahrscheinlich noch deutlich höher. Ferner ist hier zu berücksichtigen, dass der Katalysator über den untersuchten Zeitraum hinaus vermutlich noch sehr viel länger stabil ist, wodurch dieser Wert wiederum entsprechend ansteigen kann.

### Beispiel 4

### Selektive Oxidation von Lactose mit Gold-Katalysatoren

Lactose ist ein 1,4 verknüpftes Disaccharid, das aus einem Glucose- und einem Galactose-Teil besteht. Zur Oxidation von Lactose wurde der 0,5 % Au/TiO₂ (Katalysator G) eingesetzt. Zum Vergleich wurden auch der 5 % Pt/Al₂O₃-Katalysator (Engelhard) und der 5 % Pd/Al₂O₃-Katalysator eingesetzt. Die Oxidation von Lactose erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 10 mmol/l |
| pH-Wert: | 8 |
| Temperatur: | 80°C |
| Druck: | 1 bar |
| O₂-Begasungsrate: | 500 ml/min |
| Rührergeschwindigkeit: | 700 UpM |

Tabelle 7 zeigt die unter Verwendung der verschiedenen Katalysatoren erhaltenen Ergebnisse der katalytischen Lactose-Oxidation.

**Tabelle 7: Verwendung unterschiedlicher Katalysatoren zur Lactose-Oxidation, Anfangsaktivität bis 10% Umsatz**

| **Eigenschaft** | **5 %** | **5 %** | **0,5 %** |
|---|---|---|---|
| | **Pt/Al₂O₃** | **Pd/Al₂O₃** | **Au/TiO₂** |
| Umsatz nach 20 | <40 | <60 | >95 |
| min in %* | | | |
| Anfangsaktivität | 7,1 | 16,4 | 143 |
| in mmol_{Substrat}/ | | | |
| g_{Metall·}min) * | | | |

| | | | |
|---|---|---|---|
| * abgeschätzt aus Titrationskurve | | | |

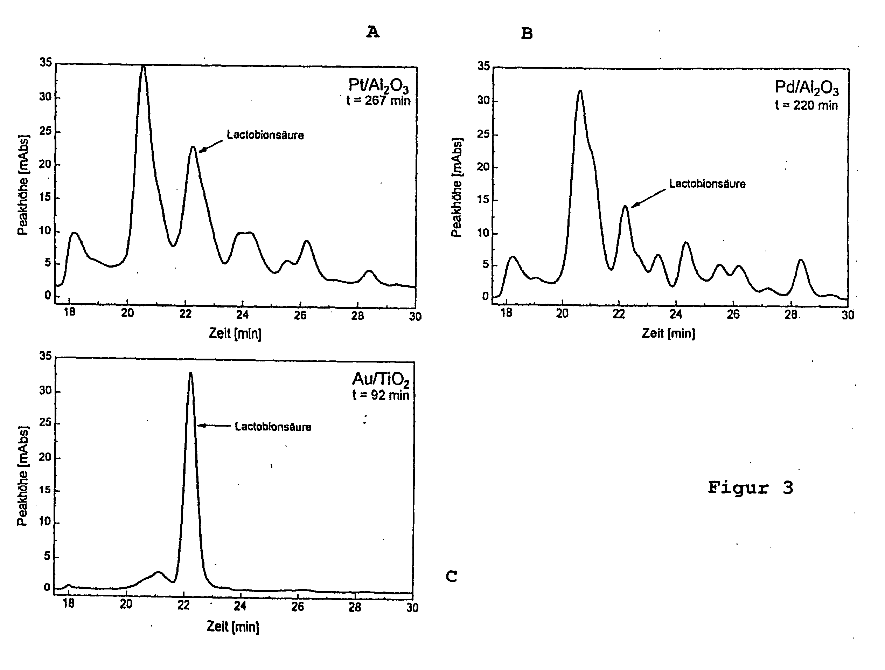

Aufgrund analytischer Probleme kann für die Lactose-Oxidation die Selektivität der einzelnen Katalysatoren nur qualitativ angegeben werden. Dazu lassen sich die im UV-Detektor erhaltenen, in Figur 3 dargestellten Chromatogramme heranziehen, die die oxidierten Reaktionsprodukte zeigen. Aus Figur 3 ist zu ersehen, dass der erfindungsgemäß eingesetztte TiO₂-geträgerte Gold-Katalysator eine extrem hohe Selektivität bezüglich der Herstellung von Lactobionsäure besitzt, wobei praktisch keine anderen Oxidationsprodukte nachzuweisen sind. Im Gegensatz dazu führt die Oxidation von Lactose in Gegenwart des Platin- beziehungsweise Palladium-Katalysators zu einem Gemisch unterschiedlicher Produkte, wobei Lactobionsäure nicht das Hauptprodukt darstellt.

### Beispiel 5:

### Selektive Oxidation von Maltose unter Verwendung von Gold-Katalysatoren

Maltose ist ein 1,4-verknüpftes Disaccharid, das aus zwei Glucoseeinheiten besteht. Dabei verfügt ein Glucose-Teil über einen aldehydischen C1-Kohlenstoff. Beide Glucoseeinheiten von Maltose enthalten jeweils ein alkoholisches C6-Kohlenstoffatom. Die Oxidation von Maltose wurde unter Verwendung des 0,5 % Au/TiO₂-Katalysators (Katalysator G) durchgeführt. Zum Vergleich erfolgte die Maltose-Oxidation auch unter Verwendung des 5 % Pt/Al₂O₂-Katalysators (Engelhard) und des 5 % Pd/Al₂O₃-Katalysators. Die Maltose-Oxidation erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 10 mol/l |
| pH-Wert: | 8 |
| Temperatur: | 80°C |
| Druck: | 1 bar |
| O₂-Begasungsrate: | 500 ml/min |
| Rührergeschwindigkeit: | 700 UpM |

Tabelle 8 zeigt die bei der Maltose-Oxidation erhaltenen Ergebnisse.

**Tabelle 8: Verwendung unterschiedlicher Katalysatoren zur Maltose-Oxidation, Anfangsaktivität bis 10% Umsatz**

| **Eigenschaft** | **5 %** | **5 %** | **0,5 %** |
|---|---|---|---|
| | **Pt/Al₂O₃** | **Pd/Al₂O₃** | **Au/TiO₂** |
| Umsatz nach 20 | <80 | 100 | 100 |
| min in %* | | | |
| Anfangsaktivität | 12,3 | 22,2 | 110 |
| in mmol_{Substrat}/ | | | |
| g_{Metall}·min)* | | | |

| | | | |
|---|---|---|---|
| * abgeschätzt aus Titrationskurve | | | |

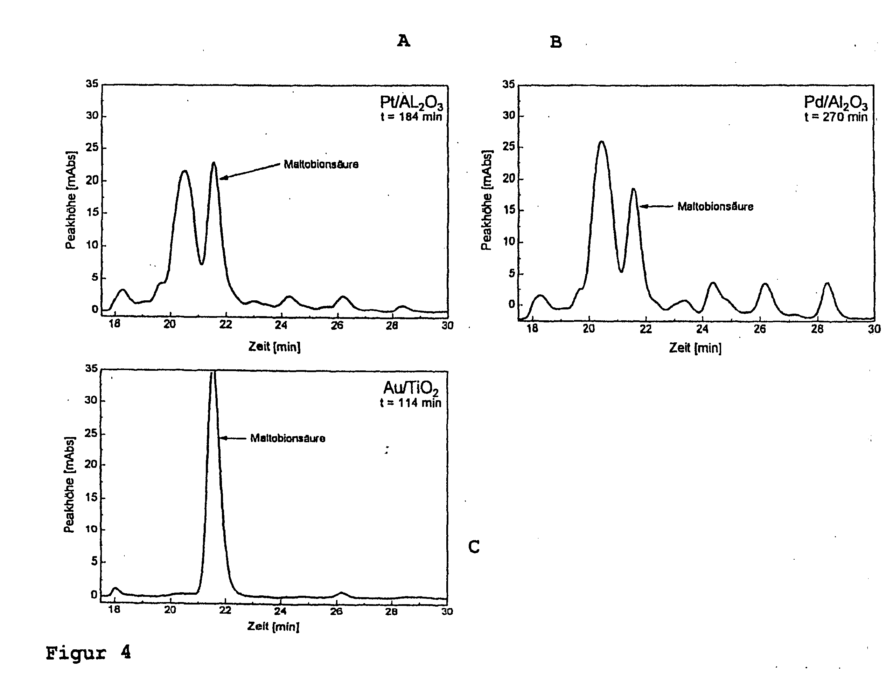

Aufgrund analytischer Probleme kann die Selektivität bezüglich der Oxidation von Maltose zu Maltobionsäure nur qualitativ angegeben werden. Dazu lassen sich die in Figur 4 dargestellten Produkt-Chromatogramme im UV-Detektor heranziehen, anhand derer die oxidierten Reaktionsprodukte nachweisbar sind. Figur 4 zeigt Chromatogramme der bei der Maltose-Oxidation unter Verwendung der angegebenen Katalysatoren erhaltenen Produkte. Aus Figur 4 ist zu ersehen, dass der verwendete Gold-Katalysator im Vergleich zu den Platin- und Palladium-Katalysatoren eine wesentlich höhere Selektivität hinsichtlich der Herstellung von Maltobionsäure besitzt, wobei praktisch keine anderen Oxidationsprodukte nachzuweisen sind. Hingegen werden bei dem Platin- beziehungsweise Palladium-Katalysator auch andere Oxidationsprodukte mit hoher Selektivität gebildet. Diese Ergebnisse zeigen, dass der verwendete Au/TiO₂-Katalysator eine ebenso hohe Selektivität bei der Maltoseoxidation zeigt wie bei der Lactose-Oxidation.

### Beispiel 6:

### Oxidation von Maltodextrin unter Verwendung eines Gold-Katalysators

Maltodextrine stellen Gemische von Oligosacchariden dar, die aus der Verknüpfung von GlucoseEinheiten über eine 1,4-glycosidische Bindung entstehen. Für das Beispiel wurde Agenamalt 20.222 (Maltodextrin DE 19) verwendet. Gemäß Angaben des Herstellers ist das verwendete Maltodextrin DE 19 wie folgt zusammengesetzt:

| | |
|---|---|
| Glucose: | 3,5-4,5 % i.d.TM |
| Maltose: | 3,5-4,5 % i.d.TM |
| Maltotriose: | 4,5-5,5 % i.d.TM |
| Oligosaccharide: | Rest |

Zur Maltodextrin-Oxidation wurden der 5% Pt/Al₂O₃-Katalysator (Engelhard), der 5% Pd/Al₂O₃-Katalysator und der 0,5% Au/TiO₂-Katalysator (Katalysator G) eingesetzt. Die Maltodextrin-Oxidation erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 10 mmol/l |
| pH-Wert: | 8 |
| Temperatur: | 80°C |
| Druck: | 1 bar |
| O₂-Begasungsrate: | 500 ml/min |
| Rührergeschwindigkeit: | 700 UpM |

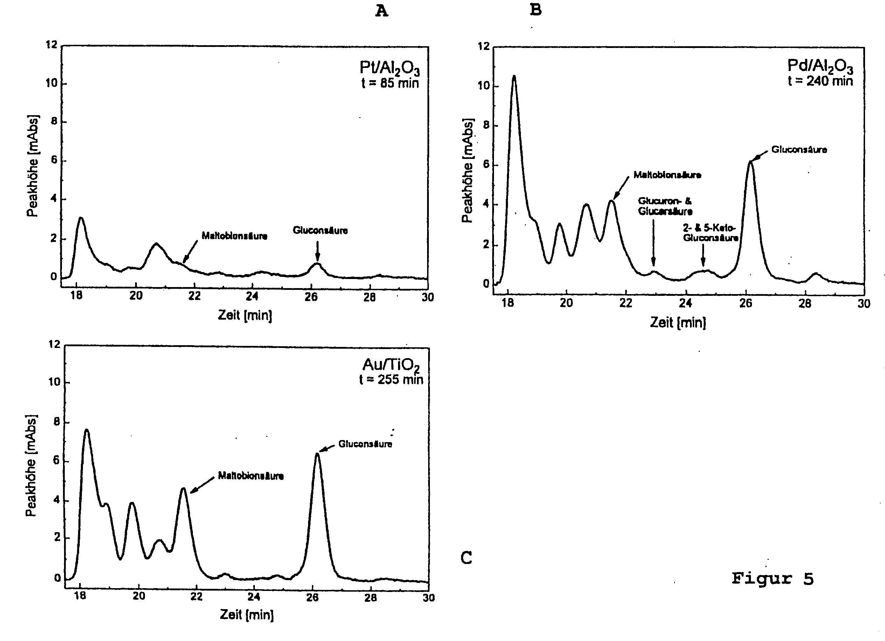

Aufgrund gravierender analytischer Probleme-können weder Umsatz noch Aktivität für die einzelnen Katalysator-Typen angegeben werden. Dennoch läuft mit jedem der untersuchten Katalysatoren eine Oxidations-Reaktion ab. Figur 5 zeigt einen qualitativen Vergleich der einzelnen UV-Chromatogramme der Proben bei Reaktionsabbruch.

Aus Figur 5 geht hervor, dass eine beachtliche Oxidation insbesondere mit dem Pd- und dem erfindungsgemäß eingesetzten Au-Katalysator abläuft, was auch durch die Menge an titrierter KOH unterstützt wird (Pt: 0,7 ml; Pd: 2,8 ml; Au: 2,2 ml jeweils bei t = 85 min). Der Vergleich des Produktgemisches, das unter Verwendung des Pd-Katalysators erhalten wurde, mit dem Produktgemisch, das unter Verwendung des Au-Katalysators erhalten wurde, zeigt einen auffälligen Unterschied. Die Substanz mit der Retentionszeit von 20,2 min wird vom Pd-Katalysator mit deutlich höherer Selektivität gebildet als dies beim Au-Katalysator der Fall ist. Die Identität dieser Substanz ist jedoch unbekannt. Hingegen zeigt der erfindungsgemäße Gold-Katalysator eine deutlich höhere Selektivität bezüglich Gluconsäure und Maltobionsäure als der Pd-Katalysator.

### Beispiel 7

### Herstellung von Gold-Katalysatoren zur selektiven Oxidation von Kohlenhydraten

### Herstellung eines Au/TiO₂-Katalysators (0,45 % Au)

Als Trägermaterial wurde ein anatas-haltiges TiO₂-Hydrat (Kronos, S_{BET} = 288 m²/g) eingesetzt. Zu einer wässrigen Suspension von 50 g TiO₂ in einem Liter destilliertem Wasser, die auf 70°C erwärmt und mit 0,2 N NaOH auf einen pH-Wert von 6,5 eingestellt wurde, werden bei konstantem pH-Wert unter intensivem Rühren innerhalb von 3 h 500 mg Tetrachlorogoldsäure (HAuCl₄ x 3 H₂O) in 250 ml Wasser zugetropft. Der Ansatz wird bei 70°C eine Stunde weiter gerührt. Nach Abkühlung auf Raumtemperatur wird eine Magnesiumcitrat-Lösung (2,318 g MgHC₆H₅O₇ x 5 H₂O in 50 ml Wasser), deren pH-Wert zuvor mit 0,2 N NaOH auf 6,5 eingestellt wurde, zugegeben. Nach einer 1-stündigen Rührzeit wird der Feststoff abzentrifugiert, dreimal mit Wasser gewaschen und anschließend im Vakuumtrockenschrank bei einem Druck von <50 hPa 17 h bei Raumtemperatur und 4 h bei 50°C getrocknet. Der erhaltene Präcursor wird leicht gemörsert und in Luft mit einer Aufheizrate von 1 K/min auf 250°C erhitzt und 3 h bei dieser Temperatur aktiviert.

Ausbeute: 47,3 g

TEM: dominierend Teilchen mit d<5 nm, vereinzelte Teilchen mit d von etwa 20 nm

ICP-OES-Analyse: 0,45 %

### Herstellung eines Au/TiO₂-Katalysators (3 % Au)

Als Trägermaterial wurde TiO₂-P25 (Degussa) eingesetzt, das aus 70 % Anatas und 30 % Rutil (S_{BET} = 50 m²/g) besteht.

Zu einer wässrigen Suspension aus 20 g TiO₂ in 400 ml destilliertem Wasser, die auf 70°C erwärmt und mit 0,2 N NaOH auf einen pH-Wert von 6,5 eingestellt wurde, werden unter intensivem Rühren bei konstantem pH-Wert innerhalb von 2,5 h 1,32 g Tetrachlorogoldsäure (HAuCl₄ x 3 H₂O) in 200 ml Wasser zugetropft. Der Ansatz wird 1 h bei 70°C gerührt. Nach Abkühlung wird eine Magnesiumcitratlösung (6,118 g MgHC₆H₅O₇ x 5 H₂O in 100 ml Wasser) zugegeben, deren pH-Wert zuvor mit 0, 2 N NaOH auf 6,5 eingestellt wurde. Nach einer 1-stündigen Rührzeit wird der Feststoff durch Zentrifugieren abgetrennt, dreimal mit Wasser gewaschen und im Vakuumtrockenschrank bei einem Druck von <50 hPa 16 h bei Raumtemperatur und 4 h bei 50°C getrocknet und anschließend leicht zermörsert. Der erhaltene Präcursor wird in Luft mit einer Aufheizrate von 1 K/min auf 250°C erhitzt und 3 h bei dieser Temperatur aktiviert.

Ausbeute: 20,5 g

TEM: Regelmäßig verteilte kleine Teilchen mit d = 1-4 nm

ICP-OES-Analyse: Au-Gehalt = 3,03 %

### Herstellung eines Au/TiO₂-Katalysators (1 % Au)

Als Trägermaterial wurde ein anatas-haltiges TiO₂-Hydrat (Kronos) verwendet, das vor seiner Belegung mit einer Polymer-stabilisierten Goldsol-Lösung 5 h bei 400°C calciniert wurde (S_{BET} = 130 m²/g).

Zu einer Lösung, bestehend aus 80 mg Tetrachlorogoldsäure (HAuCl₄ x 3 H₂O) in 400 ml Wasser, wurden 2 ml 0,1 N NaOH getropft. Unter starkem Rühren erfolge die Zugabe des kolloidstabilisierenden Polymers (120 mg Polydiallyldimethylammoniumchlorid, MG = 100000 bis 200000, 20 %ig in Wasser, verdünnt mit 4 ml Wasser). Nach Reduktion der Gold (III)-Ionen mit Natriumborhydrid (76 mg NaBH₄ in 4 ml Wasser) wurden sofort 4 g des vorbereiteten TiO₂ unter starkem Rühren hinzugefügt. Nach einstündigem Rühren wurde der Katalysator durch Zentrifugieren abgetrennt, dreimal, mit Wasser gewaschen und im Vakuumtrockenschrank bei einem Druck von <50 hPa 17 h bei Raumtemperatur und 4 h bei 50°C getrocknet.

Ausbeute: 4 g

ICP-OES-Analyse: Au-Gehalt = 1 %

### Herstellung eines Au/Al₂O₃-Katalysators (0,95 % Au)

Als Al₂O₃-Trägermaterial wurde Puralox HP 14/150 (Sasol/Condea) mit S_{BET} = 151 m²/g verwendet.

Zu einer wässrigen Suspension von 30 g Al₂O₃ in 600 ml destilliertem Wasser, die auf 70°C erwärmt und mit 0,1 N NaOH auf einen pH-Wert von 7 eingestellt wurde, werden unter intensivem Rühren innerhalb von 3 h bei konstantem pH-Wert 900 mg Tetrachlorogoldsäure (HAuCl₄ x 3 H₂O) in 450 ml Wasser zugetropft. Die Reaktionslösung wird 1 h bei 70°C weitergerührt. Zu dem auf Raumtemperatur abgekühlten Ansatz wird eine Magnesiumcitratlösung (4,172 g MgHC₆H₅O₇ x 5 H₂O in 90 ml Wasser) gegeben, deren pH-Wert zuvor mit verdünnter NaOH auf 7 eingestellt worden war. Nach 1-stündigem Rühren wird der Feststoff durch Zentrifugieren abgetrennt, dreimal mit Wasser gewaschen und im Vakuumtrockenschrank bei einem Druck von <50 hPa 17 h bei Raumtemperatur und 4 h bei 50°C getrocknet und anschließend leicht gemörsert. Der erhaltene Präcursor wird in Luft mit einer Aufheizrate von 1 K/min auf 250°C erhitzt und 3 h bei dieser Temperatur aktiviert.

Ausbeute: 27,3 g

TEM: dominierend Teilchen mit d = 1-2 nm ICP-OES-Analyse: Au-Gehalt =0,95 %

## Patentansprüche

1. Verfahren zur selektiven Oxidation von mindestens einem Kohlenhydrat, einem Kohlenhydrat-Gemisch oder einer diese(s) enthaltenden Zusammensetzung, wobei eine wässrige Lösung des Kohlenhydrates, des Gemisches oder der Zusammensetzung in Gegenwart eines Gold-Katalysators, umfassend nanodispers verteilte Gold-Partikel auf einem Metalloxid-Träger, und von Sauerstoff umgesetzt wird, wobei eine Aldehyd-Gruppe am C1-Kohlenstoffatom des/der Kohlenhydrate(s) selektiv zu einer Carboxyl-Gruppe oxidiert oder eine Aldehyd-Gruppe am C1-Kohlenstoffatom einer 2-Ketose eingeführt und selektiv zu einer Carboxyl-Gruppe oxidiert wird.

2. Verfahren nach Anspruch 1, wobei der Metalloxid-Träger des Gold-Katalysators ein TiO₂-Träger ist.

3. Verfahren nach Anspruch 2, wobei der TiO₂-geträgerte Gold-Katalysator etwa 0,1 % bis 5 % Gold, vorzugsweise etwa 0,5 % bis 1 % Gold enthält.

4. Verfahren nach Anspruch 1, wobei der Metalloxid-Träger des Gold-Katalysators ein Al₂O₃-Träger ist.

5. Verfahren nach Anspruch 4, wobei der Al₂O₃-geträgerte Gold-Katalysator etwa 0,1 % bis 5 % Gold, vorzugsweise etwa 0,5 % bis 1 % Gold enthält.

6. Verfahren nach einem der Ansprüche 1 bis 5, wobei die Oxidation bei einem pH-Wert von 7 bis 11 durchgeführt wird.

7. Verfahren nach einem der Ansprüche 1 bis 6, wobei die Oxidation bei einer Temperatur von 20°C bis 140°C, vorzugsweise 40°C bis 90°C durchgeführt wird.

8. Verfahren nach einem der Ansprüche 1 bis 7, wobei die Oxidation bei einem Druck von 1 bar bis 25 bar durchgeführt wird.

9. Verfahren nach einem der Ansprüche 1 bis 8, wobei während der Oxidation Sauerstoff und/oder Luft durch die wässrige Lösung des Kohlenhydrates, des Gemisches oder der Zusammensetzung hindurchgeperlt wird.

10. Verfahren nach einem der Ansprüche 1 bis 9, wobei das Verhältnis zwischen der Menge des/der zu oxidierenden Kohlenhydrate(s) oder des Gemisches und der Menge des auf dem Metalloxid-Trägers enthaltenen Goldes größer als 1000 ist.

11. Verfahren nach einem der Ansprüche 1 bis 10, wobei das zu oxidierende Kohlenhydrat eine Aldose mit einer Aldehyd-Gruppe am C1-Kohlenstoffatom ist.

12. Verfahren nach einem der Ansprüche 1 bis 10, wobei das zu oxidierende Kohlenhydrat in 2-Ketose-Form vorliegt, die zunächst in die oxidierbare tautomere Aldose-Form überführt wird.

13. Verfahren nach Anspruch 11 oder 12, wobei das zu oxidierende Kohlenhydrat ein Monosaccharid, ein Oligosaccharid, ein Gemisch davon oder eine diese(s) enthaltende Zusammensetzung ist.

14. Verfahren nach einem der Ansprüche 11 bis 13, wobei das zu oxidierende Monosaccharid Glucose, Galactose, Mannose, Xylose oder Ribose ist.

15. Verfahren nach Anspruch 14, wobei bei der Glucose-Oxidation als Oxidationsprodukt Gluconsäure erhalten wird.

16. Verfahren nach Anspruch 13, wobei das zu oxidierende Oligosaccharid ein Disaccharid ist.

17. Verfahren nach Anspruch 16, wobei das Disaccharid eine Disaccharid-Aldose wie Maltose, Lactose, Cellobiose oder Isomaltose ist.

18. Verfahren nach Anspruch 17, wobei bei der Maltose-Oxidation als Oxidationsprodukt Maltobionsäure erhalten wird.

19. Verfahren nach Anspruch 17, wobei bei der Lactose-Oxidation als Oxidationsprodukt Lactobionsäure erhalten wird.

20. Verfahren nach Anspruch 16, wobei das Disaccharid eine Dissaccharid-2-Ketose wie Palatinose ist.

21. Verfahren nach Anspruch 13, wobei das zu oxidierende Kohlenhydrat Maltodextrin ist.

22. Verfahren nach Anspruch 13, wobei das zu oxidierende Kohlenhydrat ein Stärkesirup ist.

23. Verwendung eines Gold-Katalysators, umfassend nanodispers verteilte Gold-Partikel auf einem Metalloxid-Träger, zur selektiven Oxidation von mindestens einem Kohlenhydrat, einem Kohlenhydrat-Gemisch oder einer diese(s) enthaltenden Zusammensetzung zu den entsprechenden Aldonsäuren.

24. Verwendung nach Anspruch 23, wobei der Metalloxid-Träger des Gold-Katalysators ein TiO₂-Träger ist.

25. Verwendung nach Anspruch 24, wobei der TiO₂-geträgerte Gold-Katalysator etwa 0,1 % bis 5 % Gold, vorzugsweise etwa 0,5 % bis 1 % Gold enthält.

26. Verwendung nach Anspruch 23, wobei der Metalloxid-Träger des Gold-Katalysators ein Al₂O₃-Träger ist.

27. Verwendung nach Anspruch 26, wobei der Al₂O₃-geträgerte Gold-Katalysator etwa 0,1 % bis 5 % Gold, vorzugsweise etwa 0,5 % bis 1 % Gold enthält.

28. Verwendung nach einem der Ansprüche 23 bis 27, wobei das zu oxidierende Kohlenhydrat ein Monosaccharid, ein Oligosaccharid, ein Gemisch davon oder eine diese(s) enthaltende Zusammensetzung ist.

29. Verwendung nach Anspruch 28, wobei das zu oxidierende Monosaccharid Glucose, Galactose, Mannose, Xylose oder Ribose ist.

30. Verwendung nach Anspruch 29, wobei als Glucose-Oxidationsprodukt Gluconsäure erhalten wird.

31. Verwendung nach Anspruch 28, wobei das zu oxidierende Oligosaccharid eine Disaccharid-Aldose ist.

32. Verwendung nach Anspruch 31, wobei die zu oxidierende Disaccharid-Aldose Maltose, Lactose, Cellobiose oder Isomaltose ist.

33. Verwendung nach Anspruch 32, wobei als Maltose-Oxidationsprodukt Maltobionsäure erhalten wird.

34. Verwendung nach Anspruch 32, wobei als Lactose-Oxidationsprodukt Lactobionsäure erhalten wird.

35. Verwendung nach Anspruch 28, wobei das zu oxidierende Oligosaccharid eine Disaccharid-Ketose ist.

36. Verwendung nach Anspruch 35, wobei die zu oxidierende Disaccharid-Ketose Palatinose ist.

37. Verwendung nach Anspruch 28, wobei das zu oxidierende Kohlenhydrat Maltodextrin ist.

38. Verwendung nach Anspruch 28, wobei das zu oxidierende Kohlenhydrat ein Stärkesirup ist.

## Claims

1. A method for selective oxidation of at least one carbohydrate, a mixture of carbohydrates or a composition containing these or either of these, wherein an aqueous solution of the carbohydrate, mixture or composition is reacted in the presence of a gold catalyst, which comprises nanodispersed gold particles on a metal oxide support, and oxygen, wherein an aldehyde group on the C1 carbon atom of the carbohydrate(s) is selectively oxidized to form a carboxyl group or an aldehyde group on the C1 carbon atom of a 2-ketose is introduced and selectively oxidized to form a carboxyl group.

2. Method according to claim 1, wherein the metal oxide support of the gold catalyst is a TiO₂ support.

3. Method according to claim 2, wherein the TiO₂-supported gold catalyst contains approx. 0.1 % to 5 % gold, preferably approx. 0.5 % to 1 % gold.

4. Method according to claim 1, wherein the metal oxide support of the gold catalyst is an Al₂O₃ support.

5. Method according to claim 4, wherein the Al₂O₃-supported gold catalyst contains approx. 0.1 % to 5 % gold, preferably approximately 0.5 % to 1 % gold.

6. Method according to any one of the claims 1 to 5, wherein the oxidation is carried out at a pH from 7 to 11.

7. Method according to any one of the claims 1 to 6, wherein the oxidation is carried out at a temperature from 20°C to 140°C, preferably 40°C to 90°C.

8. Method according to any one of the claims 1 to 7, wherein the oxidation is carried out at a pressure from 1 bar to 25 bar.

9. Method according to any one of the claims 1 to 8, wherein oxygen and/or air is bubbled through the aqueous solution of the carbohydrate, mixture of composition during the oxidation.

10. Method according to any one of the claims 1 to 9, wherein the ratio of the amount of carbohydrate(s) or mixture to be oxidized and the amount of gold contained on the metal oxide support is larger than 1,000.

11. Method according to any one of the claims 1 to 10, wherein the carbohydrate to be oxidized is an aldose having an aldehyde group on the C1 carbon atom.

12. Method according to any one of the claims 1 to 10, wherein the carbohydrate to be oxidized is present in the form of a 2-ketose that is initially converted into the oxidizable tautomeric aldose form.

13. Method according to claim 11 or 12, wherein the carbohydrate to be oxidized is a monosaccharide, an oligosaccharide, a mixture thereof or a composition containing these or either of these.

14. Method according to any one of the claims 11 to 13, wherein the monosaccharide to be oxidized is glucose, galactose, mannose, xylose or ribose.

15. Method according to claim 14, wherein gluconic acid is obtained as oxidation product during glucose oxidation.

16. Method according to claim 13, wherein the oligosaccharide to be oxidized is a disaccharide.

17. Method according to claim 16, wherein the disaccharide is a disaccharide-aldose such as maltose, lactose, cellobiose or isomaltose.

18. Method according to claim 17, wherein maltobionic acid is obtained as oxidation product during maltose oxidation.

19. Method according to claim 17, wherein lactobionic acid is obtained as oxidation product during lactose oxidation.

20. Method according to claim 16, wherein the disaccharide is a disaccharide-2-ketose such as palatinose.

21. Method according to claim 13, wherein the carbohydrate to be oxidized is maltodextrin.

22. Method according to claim 13, wherein the carbohydrate to be oxidized is a starch syrup.

23. Use of a gold catalyst comprising nanodispersed gold particles on a metal oxide support for selective oxidation of at least one carbohydrate, a mixture of carbohydrates or a composition containing these or either of these to form the corresponding aldonic acids.

24. Use according to claim 23, wherein the metal oxide support of the gold catalyst is a TiO₂ support.

25. Use according to claim 24, wherein the TiO₂-supported gold catalyst contains approx. 0.1 % to 5 % gold, preferably approx. 0.5 % to 1 % gold.

26. Use according to claim 23, wherein the metal oxide support of the gold catalyst is an Al₂O₃ support.

27. Use according to claim 26, wherein the Al₂O₃-supported gold catalyst contains approx. 0.1 % to 5 % gold, preferably approximately 0.5 % to 1 % gold.

28. Use according to any one of the claims 23 to 27, wherein the carbohydrate to be oxidized is a monosaccharide, an oligosaccharide, a mixture thereof or a composition containing these or either of these.

29. Use according to claim 28, wherein the monosaccharide to be oxidized is glucose, galactose, mannose, xylose or ribose.

30. Use according to claim 29, wherein gluconic acid is obtained as glucose oxidation product.

31. Use according to claim 28, wherein the oligosaccharide to be oxidized is a disaccharide-aldose.

32. Use according to claim 31, wherein the disaccharide-aldose to be oxidized is maltose, lactose, cellobiose or isomaltose.

33. Use according to claim 32, wherein maltobionic acid is obtained as the maltose oxidation product.

34. Use according to claim 32, wherein lactobionic acid is obtained as the lactose oxidation product.

35. Use according to claim 28, wherein the oligosaccharide to be oxidized is a disaccharide-ketose.

36. Use according to claim 35, wherein the disaccharide-ketose to be oxidized is palatinose.

37. Use according to claim 28, wherein the carbohydrate to be oxidized is maltodextrin.

38. Use according to claim 28, wherein the carbohydrate to be oxidized is a starch syrup.

## Revendications

1. Procédé pour l'oxydation sélective d'au moins un glucide, un mélange de glucides ou d'une composition contenant celui-ci ou ceux-ci, dans lequel une solution aqueuse du glucide, du mélange ou de la composition est transformée en présence d'un catalyseur d'or comprenant des particules d'or réparties de manière nanodispersée sur un support en oxyde métallique, et d'oxygène, dans lequel un groupe aldéhyde sur l'atome de carbone C1 du/des glucide(s) est oxydé de manière sélective en un groupe carboxyle ou un groupe aldéhyde sur l'atome de carbone C1 d'un 2-cétose est introduit et oxydé de manière sélective en un groupe carboxyle.

2. Procédé selon la revendication 1, dans lequel le support en oxyde métallique du catalyseur d'or est un support en TiO₂.

3. Procédé selon la revendication 2, dans lequel le catalyseur d'or à support en TiO₂ contient environ 0,1 % à 5 % d'or, de préférence environ 0,5 % à 1 % d'or.

4. Procédé selon la revendication 1, dans lequel le support en oxyde métallique du catalyseur d'or est un support en Al₂O₃.

5. Procédé selon la revendication 4, dans lequel le catalyseur d'or à support en Al₂O₃ contient environ 0,1 % à 5 % d'or, de préférence environ 0,5 % à 1 % d'or.

6. Procédé selon l'une quelconque des revendications 1 à 5, dans lequel l'oxydation est réalisée à un pH de 7 à 11.

7. Procédé selon l'une quelconque des revendications 1 à 6, dans lequel l'oxydation est réalisée à une température de 20°C à 140°C, de préférence 40°C à 90°C.

8. Procédé selon l'une quelconque des revendications 1 à 7, dans lequel l'oxydation est réalisée à une pression de 1 bar à 25 bars.

9. Procédé selon l'une quelconque des revendications 1 à 8, dans lequel de l'oxygène et/ou de l'air subit un barbotage à travers la solution aqueuse du glucide, du mélange ou de la composition pendant l'oxydation.

10. Procédé selon l'une quelconque des revendications 1 à 9, dans lequel le rapport entre la quantité du/des glucide(s) à oxyder ou du mélange et la quantité de l'or contenu sur le support en oxyde métallique est supérieur à 1000.

11. Procédé selon l'une quelconque des revendications 1 à 10, dans lequel le glucide à oxyder est un aldose avec un groupe aldéhyde sur l'atome de carbone C1.

12. Procédé selon l'une quelconque des revendications 1 à 10, dans lequel le glucide à oxyder se présente sous la forme d'un 2-cétose qui est d'abord transformé en la forme d'aldose tautomère oxydable.

13. Procédé selon la revendication 11 ou 12, dans lequel le glucide à oxyder est un monosaccharide, un oligosaccharide, un mélange de ceux-ci ou une composition contenant celui-ci ou ceux-ci.

14. Procédé selon l'une quelconque des revendications 11 à 13, dans lequel le monosaccharide à oxyder est le glucose, galactose, mannose, xylose ou ribose.

15. Procédé selon la revendication 14, dans lequel de l'acide gluconique est obtenu en tant que produit d'oxydation lors de l'oxydation de glucose.

16. Procédé selon la revendication 13, dans lequel l'oligosaccharide à oxyder est un disaccharide.

17. Procédé selon la revendication 16, dans lequel le disaccharide est un disaccharide-aldose comme le maltose, lactose, cellobiose ou isomaltose.

18. Procédé selon la revendication 17, dans lequel de l'acide maltobionique est obtenu en tant que produit d'oxydation lors de l'oxydation de maltose.

19. Procédé selon la revendication 17, dans lequel de l'acide lactobionique est obtenu en tant que produit d'oxydation lors de l'oxydation de lactose.

20. Procédé selon la revendication 16, dans lequel le disaccharide est un disaccharide-2-cétose comme le palatinose.

21. Procédé selon la revendication 13, dans lequel le glucide à oxyder est la maltodextrine.

22. Procédé selon la revendication 13, dans lequel le glucide à oxyder est un sirop d'amidon.

23. Utilisation d'un catalyseur d'or comprenant des particules d'or réparties de manière nanodispersée sur un support en oxyde métallique pour l'oxydation sélective d'au moins un glucide, un mélange de glucides ou une composition contenant celui-ci ou ceux-ci en les acides aldoniques correspondants.

24. Utilisation selon la revendication 23, dans laquelle le support en oxyde métallique du catalyseur d'or est un support en TiO₂.

25. Utilisation selon la revendication 24, dans laquelle le catalyseur d'or à support en TiO₂ contient environ 0,1 % à 5 % d'or, de préférence environ 0,5 % à 1 % d'or.

26. Utilisation selon la revendication 23, dans laquelle le support en oxyde métallique du catalyseur d'or est un support en Al₂O₃.

27. Utilisation selon la revendication 26, dans laquelle le catalyseur d'or à support en Al₂O₃ contient environ 0,1 % à 5 % d'or, de préférence environ 0,5 % à 1 % d'or.

28. Utilisation selon l'une quelconque des revendications 23 à 27, dans laquelle le glucide à oxyder est un monosaccharide, un oligosaccharide, un mélange de ceux-ci ou une composition contenant celui-ci ou ceux-ci.

29. Utilisation selon la revendication 28, dans laquelle le monosaccharide à oxyder est le glucose, galactose, mannose, xylose ou ribose.

30. Utilisation selon la revendication 29, dans laquelle de l'acide gluconique est obtenu en tant que produit d'oxydation du glucose.

31. Utilisation selon la revendication 28, dans laquelle l'oligosaccharide à oxyder est un disaccharide-aldose.

32. Utilisation selon la revendication 31, dans laquelle le disaccharide-aldose à oxyder est le maltose, lactose, cellobiose ou isomaltose.

33. Utilisation selon la revendication 32, dans laquelle de l'acide maltobionique est obtenu en tant que produit d'oxydation du maltose.

34. Utilisation selon la revendication 32, dans laquelle de l'acide lactobionique est obtenu en tant que produit d'oxydation du lactose.

35. Utilisation selon la revendication 28, dans laquelle l'oligosaccharide à oxyder est un disaccharide-cétose.

36. Utilisation selon la revendication 35, dans laquelle le disaccharide-cétose à oxyder est le palatinose.

37. Utilisation selon la revendication 28, dans laquelle le glucide à oxyder est la maltodextrine.

38. Utilisation selon la revendication 28, dans laquelle le glucide à oxyder est un sirop d'amidon.
